# EUROPEAN PATENT APPLICATION

(11) **EP 1 764 113 A1**
(43) Date of publication of application: **21.03.2007**
(21) Application number: 06124360.6
(22) Date of filing: 02.03.2004
(51) Int. Cl.: A61K 47/48, C12N 15/62, C12N 15/85, A61K 39/395, A61P 35/00, C12N 5/10, C07K 19/00, C07K 16/30, C07K 14/21, C07K 14/33, C12N 15/13

(54) **Antibodies against lung cancer, antigens and methods of use thereof for treating lung cancer**

(30) Priority: 03.03.2003 US 451765 P
(62) Divisional of application: 04716190.6
(71) Applicant: UNIVERSITY OF VICTORIA INNOVATION AND DEVELOPMENT CORPORATION, Victoria, British Columbia V8W 3W2 (CA); NATIONAL RESEARCH COUNCIL OF CANADA, Ottawa, Ontario K1A 0R6 (CA)
(72) Inventor: Buckley, Thomas, J., Victoria British Columbia V8S 4K3 (CA); Mackenzie, Roger, Ottawa Ontario K1J 6K9 (CA)
(74) Representative: Fleuchaus, Andrea

(57) **Abstract**

Disclosed herein are peptides that specifically bind to a lung cancer cell, as well as nucleic acid sequences that encode such peptides.

### Annex to the application documents - subsequently filed sequences listing

SEQUENCE LISTING

<110>  National Research Council of Canada

       The University of Victoria Innovation and Development Corporation

<120>  MODIFIED AEROLYSIN MOLECULES AND METHODS OF USE FOR TREATMENT OF
LUNG CANCER

<130>  67825

<150>  US 60/451,765

<151>  2003-03-03

<160>  8

<170>  PatentIn version 3.2


<210>  1

<211>  36

<212>  DNA

<213>  artifical sequence

<400>  1

ggtgcgcagg ccgtcttcga ggtccagctg caggcg                                36


<210>  2

<211>  30

<212>  DNA

<213>  artifical sequence

<400>  2

cgtacggtgc actgaggaga cggtgacctg                                       30


<210>  3

<211>  30

<212>  DNA

<213>  artifical sequence

<400>  3

cgtacggtgc accctgttac cggtgaaata                                       30

<210> 4

<211> 29

<212> DNA

<213> artifical sequence

<400> 4

ttctgtaagc tttgattggc agcagggga 29

<210> 5

<211> 1578

<212> DNA

<213> artifical sequence

<220>

<221> CDS

<222> (1)..(1578)

<400> 5

atg aaa aaa acc gct atc gcg atc gca gtt gca ctg gct ggt ttc gct 48

Met Lys Lys Thr Ala Ile Ala Ile Ala Val Ala Leu Ala Gly Phe Ala

1               5                   10                  15

acc gtt gcg cag gcc gat gtg cag ctg cag gcg tct gga gga ggc gtg 96

Thr Val Ala Gln Ala Asp Val Gln Leu Gln Ala Ser Gly Gly Gly Val

            20                  25                  30

gtg cag cct ggg ggg tct ctg aga ctc tcc tgt gca gcc cat gat ccc 144

Val Gln Pro Gly Gly Ser Leu Arg Leu Ser Cys Ala Ala His Asp Pro

        35                  40                  45

atc ttc gat aag aat ctc atg ggc tgg ggc cgc cag gct cca gga aag 192

Ile Phe Asp Lys Asn Leu Met Gly Trp Gly Arg Gln Ala Pro Gly Lys

    50                  55                  60

cag cgc gaa tat gtc gcg act att agt ggt aat ggt gga aca aat tat 240
Gln Arg Glu Tyr Val Ala Thr Ile Ser Gly Asn Gly Gly Thr Asn Tyr
65                  70                  75                  80

gca agc tcc gtt gag ggc cga ttc acc atc tct aga gac aac gcc aag 288
Ala Ser Ser Val Glu Gly Arg Phe Thr Ile Ser Arg Asp Asn Ala Lys
                85                  90                  95

aaa acg gtg tat ctg caa atg aac gac ctg aaa cct gag gac acg gcc 336
Lys Thr Val Tyr Leu Gln Met Asn Asp Leu Lys Pro Glu Asp Thr Ala
            100                 105                 110

gtc tat tac tgt aac tca gct ttt gct atc tgg ggc cag ggc atc cag 384
Val Tyr Tyr Cys Asn Ser Ala Phe Ala Ile Trp Gly Gln Gly Ile Gln
        115                 120                 125

gtc acc gtc tcc tca gtg cac cct gtt acc ggt gaa ata ccg aca ctg 432
Val Thr Val Ser Ser Val His Pro Val Thr Gly Glu Ile Pro Thr Leu
    130                 135                 140

tct gcc ctg gat att cca gat ggt gac gaa gtc gat gtg cag tgg cga 480
Ser Ala Leu Asp Ile Pro Asp Gly Asp Glu Val Asp Val Gln Trp Arg
145                 150                 155                 160

ctg gta cat gac agt gcg aat ttc atc aaa cca acc agc tat ctg gcc 528
Leu Val His Asp Ser Ala Asn Phe Ile Lys Pro Thr Ser Tyr Leu Ala
                165                 170                 175

cat tac ctc ggt tat gcc tgg gtg ggc ggc aat cac agc caa tat gtc 576
His Tyr Leu Gly Tyr Ala Trp Val Gly Gly Asn His Ser Gln Tyr Val
            180                 185                 190

ggc gaa gac atg gat gtg acc cgt gat ggc gac ggc tgg gtg atc cgt 624
Gly Glu Asp Met Asp Val Thr Arg Asp Gly Asp Gly Trp Val Ile Arg
        195                 200                 205

ggc aac aat gac ggc ggc tgt gac ggc tat cgc tgt ggt gac aag acg 672
Gly Asn Asn Asp Gly Gly Cys Asp Gly Tyr Arg Cys Gly Asp Lys Thr
    210                 215                 220

gcc atc aag gtc agc aac ttc gcc tat aac ctg gat ccc gac agc ttc 720
Ala Ile Lys Val Ser Asn Phe Ala Tyr Asn Leu Asp Pro Asp Ser Phe
225                 230                 235                 240

aag cat ggc gat gtc acc cag tcc gac cgc cag ctg gtc aag act gtg 768
Lys His Gly Asp Val Thr Gln Ser Asp Arg Gln Leu Val Lys Thr Val
                245                 250                 255

gtg ggc tgg gcg gtc aac gac agc gac acc ccc caa tcc ggc tat gac 816
Val Gly Trp Ala Val Asn Asp Ser Asp Thr Pro Gln Ser Gly Tyr Asp
            260                 265                 270

gtc acc ctg cgc tac gac aca gcc acc aac tgg tcc aag acc aac acc 864
Val Thr Leu Arg Tyr Asp Thr Ala Thr Asn Trp Ser Lys Thr Asn Thr
        275                 280                 285

tat ggc ctg agc gag aag gtg acc acc aag aac aag ttc aag tgg cca 912
Tyr Gly Leu Ser Glu Lys Val Thr Thr Lys Asn Lys Phe Lys Trp Pro
    290                 295                 300

ctg gtg ggg gaa acc caa ctc tcc atc gag att gct gcc aat cag tcc 960
Leu Val Gly Glu Thr Gln Leu Ser Ile Glu Ile Ala Ala Asn Gln Ser
305                 310                 315                 320

tgg gcg tcc cag aac ggg ggc tcg acc acc acc tcc ctg tct cag tcc 1008
Trp Ala Ser Gln Asn Gly Gly Ser Thr Thr Thr Ser Leu Ser Gln Ser
                325                 330                 335

gtg cga ccg act gtg ccg gcc cgc tcc aag atc ccg gtg aag ata gag 1056
Val Arg Pro Thr Val Pro Ala Arg Ser Lys Ile Pro Val Lys Ile Glu

340                 345                 350

ctc tac aag gcc gac atc tcc tat ccc tat gag ttc aag gcc gat gtc      1104

Leu Tyr Lys Ala Asp Ile Ser Tyr Pro Tyr Glu Phe Lys Ala Asp Val

        355                 360                 365

agc tat gac ctg acc ctg agc ggc ttc ctg cgc tgg ggc ggc aac gcc      1152

Ser Tyr Asp Leu Thr Leu Ser Gly Phe Leu Arg Trp Gly Gly Asn Ala

    370                 375                 380

tgg tat acc cac ccg gac aac cgt ccg aac tgg aac cac acc ttc gtc      1200

Trp Tyr Thr His Pro Asp Asn Arg Pro Asn Trp Asn His Thr Phe Val

385                 390                 395                 400

ata ggt ccg tac aag gac aag gcg agc agc att cgg tac cag tgg gac      1248

Ile Gly Pro Tyr Lys Asp Lys Ala Ser Ser Ile Arg Tyr Gln Trp Asp

                405                 410                 415

aag cgt tac atc ccg ggt gaa gtg aag tgg tgg gac tgg aac tgg acc      1296

Lys Arg Tyr Ile Pro Gly Glu Val Lys Trp Trp Asp Trp Asn Trp Thr

            420                 425                 430

ata cag cag aac ggt ctg tct acc atg cag aac aac ctg gcc aga gtg      1344

Ile Gln Gln Asn Gly Leu Ser Thr Met Gln Asn Asn Leu Ala Arg Val

        435                 440                 445

ctg cgc ccg gtg cgg gcg ggg atc acc ggt gat ttc agt gcc gag agc      1392

Leu Arg Pro Val Arg Ala Gly Ile Thr Gly Asp Phe Ser Ala Glu Ser

    450                 455                 460

cag ttt gcc ggc aac ata gag atc ggt gct ccc gtg ccg ctc gcg gct      1440

Gln Phe Ala Gly Asn Ile Glu Ile Gly Ala Pro Val Pro Leu Ala Ala

465                 470                 475                 480

gac agc aag gtg cgt cgt gct cgc agt gtg gac ggc gct ggt caa ggc     1488
Asp Ser Lys Val Arg Arg Ala Arg Ser Val Asp Gly Ala Gly Gln Gly
                485                 490                 495

ctg agg ctg gag atc ccg ctc gat gcg caa gag ctc tcc ggg ctt ggc     1536
Leu Arg Leu Glu Ile Pro Leu Asp Ala Gln Glu Leu Ser Gly Leu Gly
            500                 505                 510

ttc aac aac gtc agc ctc agc gtg tcc cct gct gcc aat caa             1578
Phe Asn Asn Val Ser Leu Ser Val Ser Pro Ala Ala Asn Gln
        515                 520                 525

<210>  6
<211>  526
<212>  PRT
<213>  artifical sequence
<400>  6

Met Lys Lys Thr Ala Ile Ala Ile Ala Val Ala Leu Ala Gly Phe Ala
1               5                   10                  15

Thr Val Ala Gln Ala Asp Val Gln Leu Gln Ala Ser Gly Gly Gly Val
            20                  25                  30

Val Gln Pro Gly Gly Ser Leu Arg Leu Ser Cys Ala Ala His Asp Pro
        35                  40                  45

Ile Phe Asp Lys Asn Leu Met Gly Trp Gly Arg Gln Ala Pro Gly Lys
    50                  55                  60

Gln Arg Glu Tyr Val Ala Thr Ile Ser Gly Asn Gly Gly Thr Asn Tyr
65                  70                  75                  80

Ala Ser Ser Val Glu Gly Arg Phe Thr Ile Ser Arg Asp Asn Ala Lys
                85                  90                  95
Lys Thr Val Tyr Leu Gln Met Asn Asp Leu Lys Pro Glu Asp Thr Ala
            100                 105                 110
Val Tyr Tyr Cys Asn Ser Ala Phe Ala Ile Trp Gly Gln Gly Ile Gln
        115                 120                 125
Val Thr Val Ser Ser Val His Pro Val Thr Gly Glu Ile Pro Thr Leu
    130                 135                 140
Ser Ala Leu Asp Ile Pro Asp Gly Asp Glu Val Asp Val Gln Trp Arg
145                 150                 155                 160
Leu Val His Asp Ser Ala Asn Phe Ile Lys Pro Thr Ser Tyr Leu Ala
                165                 170                 175
His Tyr Leu Gly Tyr Ala Trp Val Gly Gly Asn His Ser Gln Tyr Val
            180                 185                 190
Gly Glu Asp Met Asp Val Thr Arg Asp Gly Asp Gly Trp Val Ile Arg
        195                 200                 205
Gly Asn Asn Asp Gly Gly Cys Asp Gly Tyr Arg Cys Gly Asp Lys Thr
    210                 215                 220
Ala Ile Lys Val Ser Asn Phe Ala Tyr Asn Leu Asp Pro Asp Ser Phe
225                 230                 235                 240

Lys His Gly Asp Val Thr Gln Ser Asp Arg Gln Leu Val Lys Thr Val
                245                 250                 255

Val Gly Trp Ala Val Asn Asp Ser Asp Thr Pro Gln Ser Gly Tyr Asp
            260                 265                 270

Val Thr Leu Arg Tyr Asp Thr Ala Thr Asn Trp Ser Lys Thr Asn Thr
        275                 280                 285

Tyr Gly Leu Ser Glu Lys Val Thr Thr Lys Asn Lys Phe Lys Trp Pro
    290                 295                 300

Leu Val Gly Glu Thr Gln Leu Ser Ile Glu Ile Ala Ala Asn Gln Ser
305                 310                 315                 320

Trp Ala Ser Gln Asn Gly Gly Ser Thr Thr Thr Ser Leu Ser Gln Ser
                325                 330                 335

Val Arg Pro Thr Val Pro Ala Arg Ser Lys Ile Pro Val Lys Ile Glu
            340                 345                 350

Leu Tyr Lys Ala Asp Ile Ser Tyr Pro Tyr Glu Phe Lys Ala Asp Val
        355                 360                 365

Ser Tyr Asp Leu Thr Leu Ser Gly Phe Leu Arg Trp Gly Gly Asn Ala
    370                 375                 380

Trp Tyr Thr His Pro Asp Asn Arg Pro Asn Trp Asn His Thr Phe Val
385                 390                 395                 400

Ile Gly Pro Tyr Lys Asp Lys Ala Ser Ser Ile Arg Tyr Gln Trp Asp

405                 410                 415

Lys Arg Tyr Ile Pro Gly Glu Val Lys Trp Trp Asp Trp Asn Trp Thr

            420                 425                 430

Ile Gln Gln Asn Gly Leu Ser Thr Met Gln Asn Asn Leu Ala Arg Val

        435                 440                 445

Leu Arg Pro Val Arg Ala Gly Ile Thr Gly Asp Phe Ser Ala Glu Ser

    450                 455                 460

Gln Phe Ala Gly Asn Ile Glu Ile Gly Ala Pro Val Pro Leu Ala Ala

465                 470                 475                 480

Asp Ser Lys Val Arg Arg Ala Arg Ser Val Asp Gly Ala Gly Gln Gly

                485                 490                 495

Leu Arg Leu Glu Ile Pro Leu Asp Ala Gln Glu Leu Ser Gly Leu Gly

            500                 505                 510

Phe Asn Asn Val Ser Leu Ser Val Ser Pro Ala Ala Asn Gln

        515                 520                 525

<210>  7

<211>  1542

<212>  DNA

<213>  Clostridium septicum

<220>

<221>  CDS

<222>  (80)..(1411)

<400>  7

tgttaataat atgttaatat tttgataaca tttattatat aataaattat ttattttaaa      60

attaaaggga gggatattt atg tca aaa aaa tct ttt gct aaa aaa gta att     112

                     Met Ser Lys Lys Ser Phe Ala Lys Lys Val Ile

                     1               5                   10

tgt aca tct atg att gca att cag tgt gcg gca gta gta cca cat gta     160

Cys Thr Ser Met Ile Ala Ile Gln Cys Ala Ala Val Val Pro His Val

            15                  20                  25

caa gct tat gca ctt aca aat ctt gaa gag ggg gga tat gca aat cat     208

Gln Ala Tyr Ala Leu Thr Asn Leu Glu Glu Gly Gly Tyr Ala Asn His

        30                  35                  40

aat aat gct tct tca att aaa ata ttt gga tat gaa gac aat gaa gat     256

Asn Asn Ala Ser Ser Ile Lys Ile Phe Gly Tyr Glu Asp Asn Glu Asp

    45                  50                  55

tta aaa gct aaa att att caa gat cca gag ttt ata aga aat tgg gca     304

Leu Lys Ala Lys Ile Ile Gln Asp Pro Glu Phe Ile Arg Asn Trp Ala

60                  65                  70                  75

aat gta gct cat tca tta gga ttt gga tgg tgc ggt gga acg gct aat     352

Asn Val Ala His Ser Leu Gly Phe Gly Trp Cys Gly Gly Thr Ala Asn

                80                  85                  90

cca aac gtt gga caa ggt ttt gaa ttt aaa aga gaa gtt ggg gca ggt     400

Pro Asn Val Gly Gln Gly Phe Glu Phe Lys Arg Glu Val Gly Ala Gly

            95                  100                 105

gga aaa gta tct tat tta tta tct gct aga tac aat cca aat gat cct     448

Gly Lys Val Ser Tyr Leu Leu Ser Ala Arg Tyr Asn Pro Asn Asp Pro

        110                 115                 120

tat gca agt gga tat cgt gca aaa gat aga ctt tct atg aaa ata tca 496
Tyr Ala Ser Gly Tyr Arg Ala Lys Asp Arg Leu Ser Met Lys Ile Ser
    125                 130                 135

aat gtt aga ttt gtt att gat aat gat tct ata aaa tta ggt aca cct 544
Asn Val Arg Phe Val Ile Asp Asn Asp Ser Ile Lys Leu Gly Thr Pro
140                 145                 150                 155

aaa gtg aaa aaa tta gca cct tta aac tct gct agt ttt gat tta ata 592
Lys Val Lys Lys Leu Ala Pro Leu Asn Ser Ala Ser Phe Asp Leu Ile
                160                 165                 170

aat gaa agt aaa act gag tct aaa tta tca aaa aca ttt aat tat aca 640
Asn Glu Ser Lys Thr Glu Ser Lys Leu Ser Lys Thr Phe Asn Tyr Thr
            175                 180                 185

act tct aaa aca gtt tct aaa aca gat aac ttt aaa ttt gga gaa aaa 688
Thr Ser Lys Thr Val Ser Lys Thr Asp Asn Phe Lys Phe Gly Glu Lys
        190                 195                 200

ata gga gta aaa aca tca ttt aaa gta ggt ctt gaa gct ata gct gac 736
Ile Gly Val Lys Thr Ser Phe Lys Val Gly Leu Glu Ala Ile Ala Asp
    205                 210                 215

agt aaa gtt gag aca agc ttt gaa ttt aat gca gaa caa ggt tgg tca 784
Ser Lys Val Glu Thr Ser Phe Glu Phe Asn Ala Glu Gln Gly Trp Ser
220                 225                 230                 235

aat aca aat agt act act gaa act aaa caa gaa agt act aca tat act 832
Asn Thr Asn Ser Thr Thr Glu Thr Lys Gln Glu Ser Thr Thr Tyr Thr
                240                 245                 250

gca aca gtt tct cca caa act aaa aag aga tta ttc cta gat gtg tta 880
Ala Thr Val Ser Pro Gln Thr Lys Lys Arg Leu Phe Leu Asp Val Leu

            255                 260                 265

gga tca caa att gat att cct tat gaa gga aaa ata tat atg gaa tac 928
Gly Ser Gln Ile Asp Ile Pro Tyr Glu Gly Lys Ile Tyr Met Glu Tyr
        270                 275                 280

gac ata gaa tta atg gga ttt tta aga tat aca gga aat gct cgt gaa 976
Asp Ile Glu Leu Met Gly Phe Leu Arg Tyr Thr Gly Asn Ala Arg Glu
    285                 290                 295

gat cat act gaa gat aga cca aca gtt aaa ctt aaa ttt ggt aaa aac 1024
Asp His Thr Glu Asp Arg Pro Thr Val Lys Leu Lys Phe Gly Lys Asn
300                 305                 310                 315

ggt atg agt gct gag gaa cat ctt aaa gat tta tat agt cat aag aat 1072
Gly Met Ser Ala Glu Glu His Leu Lys Asp Leu Tyr Ser His Lys Asn
                320                 325                 330

att aat gga tat tca gaa tgg gat tgg aaa tgg gta gat gag aaa ttt 1120
Ile Asn Gly Tyr Ser Glu Trp Asp Trp Lys Trp Val Asp Glu Lys Phe
            335                 340                 345

ggt tat tta ttt aaa aat tca tac gat gct ctt act agt aga aaa tta 1168
Gly Tyr Leu Phe Lys Asn Ser Tyr Asp Ala Leu Thr Ser Arg Lys Leu
        350                 355                 360

gga gga ata ata aaa ggc tca ttt act aac att aat gga aca aaa ata 1216
Gly Gly Ile Ile Lys Gly Ser Phe Thr Asn Ile Asn Gly Thr Lys Ile
    365                 370                 375

gta att aga gaa ggt aaa gaa att cca ctt cct gat aag aag aga aga 1264
Val Ile Arg Glu Gly Lys Glu Ile Pro Leu Pro Asp Lys Lys Arg Arg
380                 385                 390                 395

gga aaa cgt tca gta gat tct tta gat gct aga tta caa aat gaa ggt 1312

Gly Lys Arg Ser Val Asp Ser Leu Asp Ala Arg Leu Gln Asn Glu Gly
                400                 405                 410

att aga ata gaa aat att gaa aca caa gat gtt cca gga ttt aga cta 1360
Ile Arg Ile Glu Asn Ile Glu Thr Gln Asp Val Pro Gly Phe Arg Leu
            415                 420                 425

aat agc ata aca tac aat gat aaa aaa ttg ata tta att aat aat ata 1408
Asn Ser Ile Thr Tyr Asn Asp Lys Lys Leu Ile Leu Ile Asn Asn Ile
        430                 435                 440

taa ttataattta ttaaaatatg cttctctata ctttatatta atatttaaag 1461

tataaaaact aacaaaatct cacttagtag gtagaattgt ataaaaacaa atctacctac 1521

tattttttta ttatttagtc g 1542

<210> 8
<211> 443
<212> PRT
<213> Clostridium septicum

<400> 8

Met Ser Lys Lys Ser Phe Ala Lys Lys Val Ile Cys Thr Ser Met Ile
1               5                   10                  15

Ala Ile Gln Cys Ala Ala Val Val Pro His Val Gln Ala Tyr Ala Leu
            20                  25                  30

Thr Asn Leu Glu Glu Gly Gly Tyr Ala Asn His Asn Asn Ala Ser Ser
        35                  40                  45

Ile Lys Ile Phe Gly Tyr Glu Asp Asn Glu Asp Leu Lys Ala Lys Ile

50                  55                  60

Ile Gln Asp Pro Glu Phe Ile Arg Asn Trp Ala Asn Val Ala His Ser
65                  70                  75                  80

Leu Gly Phe Gly Trp Cys Gly Gly Thr Ala Asn Pro Asn Val Gly Gln
                85                  90                  95

Gly Phe Glu Phe Lys Arg Glu Val Gly Ala Gly Gly Lys Val Ser Tyr
            100                 105                 110

Leu Leu Ser Ala Arg Tyr Asn Pro Asn Asp Pro Tyr Ala Ser Gly Tyr
        115                 120                 125

Arg Ala Lys Asp Arg Leu Ser Met Lys Ile Ser Asn Val Arg Phe Val
    130                 135                 140

Ile Asp Asn Asp Ser Ile Lys Leu Gly Thr Pro Lys Val Lys Lys Leu
145                 150                 155                 160

Ala Pro Leu Asn Ser Ala Ser Phe Asp Leu Ile Asn Glu Ser Lys Thr
                165                 170                 175

Glu Ser Lys Leu Ser Lys Thr Phe Asn Tyr Thr Thr Ser Lys Thr Val
            180                 185                 190

Ser Lys Thr Asp Asn Phe Lys Phe Gly Glu Lys Ile Gly Val Lys Thr
        195                 200                 205

Ser Phe Lys Val Gly Leu Glu Ala Ile Ala Asp Ser Lys Val Glu Thr
    210                 215                 220

Ser Phe Glu Phe Asn Ala Glu Gln Gly Trp Ser Asn Thr Asn Ser Thr
225                 230                 235                 240

Thr Glu Thr Lys Gln Glu Ser Thr Thr Tyr Thr Ala Thr Val Ser Pro

                245                 250                 255

Gln Thr Lys Lys Arg Leu Phe Leu Asp Val Leu Gly Ser Gln Ile Asp

            260                 265                 270

Ile Pro Tyr Glu Gly Lys Ile Tyr Met Glu Tyr Asp Ile Glu Leu Met

        275                 280                 285

Gly Phe Leu Arg Tyr Thr Gly Asn Ala Arg Glu Asp His Thr Glu Asp

    290                 295                 300

Arg Pro Thr Val Lys Leu Lys Phe Gly Lys Asn Gly Met Ser Ala Glu

305                 310                 315                 320

Glu His Leu Lys Asp Leu Tyr Ser His Lys Asn Ile Asn Gly Tyr Ser

                325                 330                 335

Glu Trp Asp Trp Lys Trp Val Asp Glu Lys Phe Gly Tyr Leu Phe Lys

            340                 345                 350

Asn Ser Tyr Asp Ala Leu Thr Ser Arg Lys Leu Gly Gly Ile Ile Lys

        355                 360                 365

Gly Ser Phe Thr Asn Ile Asn Gly Thr Lys Ile Val Ile Arg Glu Gly

    370                 375                 380

Lys Glu Ile Pro Leu Pro Asp Lys Lys Arg Arg Gly Lys Arg Ser Val

385                 390                 395                 400

Asp Ser Leu Asp Ala Arg Leu Gln Asn Glu Gly Ile Arg Ile Glu Asn

                405                 410                 415

Ile Glu Thr Gln Asp Val Pro Gly Phe Arg Leu Asn Ser Ile Thr Tyr

            420                 425                 430

Asn Asp Lys Lys Leu Ile Leu Ile Asn Asn Ile

        435                 440

SEQUENCE LISTING

<110> University of Victoria Innovation and Development Corporation,
National Research Council of Canada

<120> Modified Aerolysin and Methods of Use for Treating Lung Cancer

<130> P10857EPPC T1

<140> EP 06124360.6 Divisional to EP 04716190.6

<141> 2004-03-02

<150> US 60/451,761

<151> 2003-03-03

<160> 10

<170> PatentIn version 3.3

<210> 1

<211> 36

<212> DNA

<213> Artificial

<220>

<223> Oligonucleotid Primer

<400> 1

ggtgcgcagg ccgtcttcga ggtccagctg caggcg 36

<210> 2

<211> 30

<212> DNA

<213> Artificial

<220>

<223> Oligonucleotid Primer

<400> 2

cgtacggtgc actgaggaga cggtgacctg 30

<210>  3

<211>  30

<212>  DNA

<213>  Artificial

<220>

<223>  Oligonucleotid Primer


<400>  3

cgtacggtgc accctgttac cggtgaaata                                        30


<210>  4

<211>  29

<212>  DNA

<213>  Artificial

<220>

<223>  Oligonucleotid Primer


<400>  4

ttctgtaagc tttgattggc agcagggga                                         29



<210>  5

<211>  1578

<212>  DNA

<213>  Artificial

<220>

<223>  AFAI-Aerolysin hybrid; AFAI is a homodimeric heavy chain antibody
from Tylopoda; Aerolysin is a channel forming toxin e.g. from Aeromonas
hydrophilia

<220>

<221>  CDS

<222>  (1)..(1578)


<400>  5

atg aaa aaa acc gct atc gcg atc gca gtt gca ctg gct ggt ttc gct        48

Met Lys Lys Thr Ala Ile Ala Ile Ala Val Ala Leu Ala Gly Phe Ala

1               5                   10                  15

acc gtt gcg cag gcc gat gtg cag ctg cag gcg tct gga gga ggc gtg 96
Thr Val Ala Gln Ala Asp Val Gln Leu Gln Ala Ser Gly Gly Gly Val
            20                  25                  30

gtg cag cct ggg ggg tct ctg aga ctc tcc tgt gca gcc cat gat ccc 144
Val Gln Pro Gly Gly Ser Leu Arg Leu Ser Cys Ala Ala His Asp Pro
        35                  40                  45

atc ttc gat aag aat ctc atg ggc tgg ggc cgc cag gct cca gga aag 192
Ile Phe Asp Lys Asn Leu Met Gly Trp Gly Arg Gln Ala Pro Gly Lys
    50                  55                  60

cag cgc gaa tat gtc gcg act att agt ggt aat ggt gga aca aat tat 240
Gln Arg Glu Tyr Val Ala Thr Ile Ser Gly Asn Gly Gly Thr Asn Tyr
65                  70                  75                  80

gca agc tcc gtt gag ggc cga ttc acc atc tct aga gac aac gcc aag 288
Ala Ser Ser Val Glu Gly Arg Phe Thr Ile Ser Arg Asp Asn Ala Lys
                85                  90                  95

aaa acg gtg tat ctg caa atg aac gac ctg aaa cct gag gac acg gcc 336
Lys Thr Val Tyr Leu Gln Met Asn Asp Leu Lys Pro Glu Asp Thr Ala
            100                 105                 110

gtc tat tac tgt aac tca gct ttt gct atc tgg ggc cag ggc atc cag 384
Val Tyr Tyr Cys Asn Ser Ala Phe Ala Ile Trp Gly Gln Gly Ile Gln
        115                 120                 125

gtc acc gtc tcc tca gtg cac cct gtt acc ggt gaa ata ccg aca ctg 432
Val Thr Val Ser Ser Val His Pro Val Thr Gly Glu Ile Pro Thr Leu
    130                 135                 140

tct gcc ctg gat att cca gat ggt gac gaa gtc gat gtg cag tgg cga 480
Ser Ala Leu Asp Ile Pro Asp Gly Asp Glu Val Asp Val Gln Trp Arg
145                 150                 155                 160

ctg gta cat gac agt gcg aat ttc atc aaa cca acc agc tat ctg gcc 528
Leu Val His Asp Ser Ala Asn Phe Ile Lys Pro Thr Ser Tyr Leu Ala
                165                 170                 175

cat tac ctc ggt tat gcc tgg gtg ggc ggc aat cac agc caa tat gtc 576
His Tyr Leu Gly Tyr Ala Trp Val Gly Gly Asn His Ser Gln Tyr Val
            180                 185                 190

ggc gaa gac atg gat gtg acc cgt gat ggc gac ggc tgg gtg atc cgt 624
Gly Glu Asp Met Asp Val Thr Arg Asp Gly Asp Gly Trp Val Ile Arg
        195                 200                 205

ggc aac aat gac ggc ggc tgt gac ggc tat cgc tgt ggt gac aag acg 672
Gly Asn Asn Asp Gly Gly Cys Asp Gly Tyr Arg Cys Gly Asp Lys Thr
    210                 215                 220

gcc atc aag gtc agc aac ttc gcc tat aac ctg gat ccc gac agc ttc 720
Ala Ile Lys Val Ser Asn Phe Ala Tyr Asn Leu Asp Pro Asp Ser Phe
225                 230                 235                 240

aag cat ggc gat gtc acc cag tcc gac cgc cag ctg gtc aag act gtg 768
Lys His Gly Asp Val Thr Gln Ser Asp Arg Gln Leu Val Lys Thr Val
                245                 250                 255

gtg ggc tgg gcg gtc aac gac agc gac acc ccc caa tcc ggc tat gac 816
Val Gly Trp Ala Val Asn Asp Ser Asp Thr Pro Gln Ser Gly Tyr Asp
            260                 265                 270

gtc acc ctg cgc tac gac aca gcc acc aac tgg tcc aag acc aac acc 864
Val Thr Leu Arg Tyr Asp Thr Ala Thr Asn Trp Ser Lys Thr Asn Thr
        275                 280                 285

tat ggc ctg agc gag aag gtg acc acc aag aac aag ttc aag tgg cca 912
Tyr Gly Leu Ser Glu Lys Val Thr Thr Lys Asn Lys Phe Lys Trp Pro
    290                 295                 300

ctg gtg ggg gaa acc caa ctc tcc atc gag att gct gcc aat cag tcc 960
Leu Val Gly Glu Thr Gln Leu Ser Ile Glu Ile Ala Ala Asn Gln Ser
305                 310                 315                 320

tgg gcg tcc cag aac ggg ggc tcg acc acc acc tcc ctg tct cag tcc 1008
Trp Ala Ser Gln Asn Gly Gly Ser Thr Thr Thr Ser Leu Ser Gln Ser
                325                 330                 335

gtg cga ccg act gtg ccg gcc cgc tcc aag atc ccg gtg aag ata gag 1056

Val Arg Pro Thr Val Pro Ala Arg Ser Lys Ile Pro Val Lys Ile Glu
            340                 345                 350

ctc tac aag gcc gac atc tcc tat ccc tat gag ttc aag gcc gat gtc 1104
Leu Tyr Lys Ala Asp Ile Ser Tyr Pro Tyr Glu Phe Lys Ala Asp Val
        355                 360                 365

agc tat gac ctg acc ctg agc ggc ttc ctg cgc tgg ggc ggc aac gcc 1152
Ser Tyr Asp Leu Thr Leu Ser Gly Phe Leu Arg Trp Gly Gly Asn Ala
    370                 375                 380

tgg tat acc cac ccg gac aac cgt ccg aac tgg aac cac acc ttc gtc 1200
Trp Tyr Thr His Pro Asp Asn Arg Pro Asn Trp Asn His Thr Phe Val
385                 390                 395                 400

ata ggt ccg tac aag gac aag gcg agc agc att cgg tac cag tgg gac 1248
Ile Gly Pro Tyr Lys Asp Lys Ala Ser Ser Ile Arg Tyr Gln Trp Asp
                405                 410                 415

aag cgt tac atc ccg ggt gaa gtg aag tgg tgg gac tgg aac tgg acc 1296
Lys Arg Tyr Ile Pro Gly Glu Val Lys Trp Trp Asp Trp Asn Trp Thr
            420                 425                 430

ata cag cag aac ggt ctg tct acc atg cag aac aac ctg gcc aga gtg 1344
Ile Gln Gln Asn Gly Leu Ser Thr Met Gln Asn Asn Leu Ala Arg Val
        435                 440                 445

ctg cgc ccg gtg cgg gcg ggg atc acc ggt gat ttc agt gcc gag agc 1392
Leu Arg Pro Val Arg Ala Gly Ile Thr Gly Asp Phe Ser Ala Glu Ser
    450                 455                 460

cag ttt gcc ggc aac ata gag atc ggt gct ccc gtg ccg ctc gcg gct 1440
Gln Phe Ala Gly Asn Ile Glu Ile Gly Ala Pro Val Pro Leu Ala Ala
465                 470                 475                 480

gac agc aag gtg cgt cgt gct cgc agt gtg gac ggc gct ggt caa ggc 1488
Asp Ser Lys Val Arg Arg Ala Arg Ser Val Asp Gly Ala Gly Gln Gly
                485                 490                 495

ctg agg ctg gag atc ccg ctc gat gcg caa gag ctc tcc ggg ctt ggc 1536

Leu Arg Leu Glu Ile Pro Leu Asp Ala Gln Glu Leu Ser Gly Leu Gly

            500                 505                 510

ttc aac aac gtc agc ctc agc gtg tcc cct gct gcc aat caa 1578

Phe Asn Asn Val Ser Leu Ser Val Ser Pro Ala Ala Asn Gln

        515                 520                 525

<210> 6

<211> 526

<212> PRT

<213> Artificial

<220>

<223> AFAI-Aerolysin hybrid; AFAI is a homodimeric heavy chain antibody
from Tylopoda; Aerolysin is a channel forming toxin (e.g. from Aeromonas
hydrophilia)

<400> 6

Met Lys Lys Thr Ala Ile Ala Ile Ala Val Ala Leu Ala Gly Phe Ala

1               5                   10                  15

Thr Val Ala Gln Ala Asp Val Gln Leu Gln Ala Ser Gly Gly Gly Val

            20                  25                  30

Val Gln Pro Gly Gly Ser Leu Arg Leu Ser Cys Ala Ala His Asp Pro

        35                  40                  45

Ile Phe Asp Lys Asn Leu Met Gly Trp Gly Arg Gln Ala Pro Gly Lys

    50                  55                  60

Gln Arg Glu Tyr Val Ala Thr Ile Ser Gly Asn Gly Gly Thr Asn Tyr

65                  70                  75                  80

Ala Ser Ser Val Glu Gly Arg Phe Thr Ile Ser Arg Asp Asn Ala Lys

                85                  90                  95

Lys Thr Val Tyr Leu Gln Met Asn Asp Leu Lys Pro Glu Asp Thr Ala

            100                 105                 110

Val Tyr Tyr Cys Asn Ser Ala Phe Ala Ile Trp Gly Gln Gly Ile Gln

        115                 120                 125

Val Thr Val Ser Ser Val His Pro Val Thr Gly Glu Ile Pro Thr Leu
    130                 135                 140

Ser Ala Leu Asp Ile Pro Asp Gly Asp Glu Val Asp Val Gln Trp Arg
145                 150                 155                 160

Leu Val His Asp Ser Ala Asn Phe Ile Lys Pro Thr Ser Tyr Leu Ala
                165                 170                 175

His Tyr Leu Gly Tyr Ala Trp Val Gly Gly Asn His Ser Gln Tyr Val
            180                 185                 190

Gly Glu Asp Met Asp Val Thr Arg Asp Gly Asp Gly Trp Val Ile Arg
        195                 200                 205

Gly Asn Asn Asp Gly Gly Cys Asp Gly Tyr Arg Cys Gly Asp Lys Thr
    210                 215                 220

Ala Ile Lys Val Ser Asn Phe Ala Tyr Asn Leu Asp Pro Asp Ser Phe
225                 230                 235                 240

Lys His Gly Asp Val Thr Gln Ser Asp Arg Gln Leu Val Lys Thr Val
                245                 250                 255

Val Gly Trp Ala Val Asn Asp Ser Asp Thr Pro Gln Ser Gly Tyr Asp
            260                 265                 270

Val Thr Leu Arg Tyr Asp Thr Ala Thr Asn Trp Ser Lys Thr Asn Thr
        275                 280                 285

Tyr Gly Leu Ser Glu Lys Val Thr Thr Lys Asn Lys Phe Lys Trp Pro
    290                 295                 300

Leu Val Gly Glu Thr Gln Leu Ser Ile Glu Ile Ala Ala Asn Gln Ser
305                 310                 315                 320

Trp Ala Ser Gln Asn Gly Gly Ser Thr Thr Thr Ser Leu Ser Gln Ser
                325                 330                 335

Val Arg Pro Thr Val Pro Ala Arg Ser Lys Ile Pro Val Lys Ile Glu
            340                 345                 350

Leu Tyr Lys Ala Asp Ile Ser Tyr Pro Tyr Glu Phe Lys Ala Asp Val
        355                 360                 365

Ser Tyr Asp Leu Thr Leu Ser Gly Phe Leu Arg Trp Gly Gly Asn Ala
    370                 375                 380

Trp Tyr Thr His Pro Asp Asn Arg Pro Asn Trp Asn His Thr Phe Val
385                 390                 395                 400

Ile Gly Pro Tyr Lys Asp Lys Ala Ser Ser Ile Arg Tyr Gln Trp Asp
                405                 410                 415

Lys Arg Tyr Ile Pro Gly Glu Val Lys Trp Trp Asp Trp Asn Trp Thr
            420                 425                 430

Ile Gln Gln Asn Gly Leu Ser Thr Met Gln Asn Asn Leu Ala Arg Val
        435                 440                 445

Leu Arg Pro Val Arg Ala Gly Ile Thr Gly Asp Phe Ser Ala Glu Ser
    450                 455                 460

Gln Phe Ala Gly Asn Ile Glu Ile Gly Ala Pro Val Pro Leu Ala Ala
465                 470                 475                 480

Asp Ser Lys Val Arg Arg Ala Arg Ser Val Asp Gly Ala Gly Gln Gly
                485                 490                 495

Leu Arg Leu Glu Ile Pro Leu Asp Ala Gln Glu Leu Ser Gly Leu Gly
            500                 505                 510

Phe Asn Asn Val Ser Leu Ser Val Ser Pro Ala Ala Asn Gln
        515                 520                 525

<210> 7
<211> 1542
<212> DNA
<213> Clostridium septicum

<220>
<221> CDS
<222> (80)..(1411)

<400> 7
   tgttaataat atgttaatat tttgataaca tttattatat aataaattat ttattttaaa 60
   attaaaggga gggatattt atg tca aaa aaa tct ttt gct aaa aaa gta att 112
   Met Ser Lys Lys Ser Phe Ala Lys Lys Val Ile
   1 5 10
   tgt aca tct atg att gca att cag tgt gcg gca gta gta cca cat gta 160
   Cys Thr Ser Met Ile Ala Ile Gln Cys Ala Ala Val Val Pro His Val
   15 20 25
   caa gct tat gca ctt aca aat ctt gaa gag ggg gga tat gca aat cat 208
   Gln Ala Tyr Ala Leu Thr Asn Leu Glu Glu Gly Gly Tyr Ala Asn His
   30 35 40
   aat aat gct tct tca att aaa ata ttt gga tat gaa gac aat gaa gat 256
   Asn Asn Ala Ser Ser Ile Lys Ile Phe Gly Tyr Glu Asp Asn Glu Asp
   45 50 55
   tta aaa gct aaa att att caa gat cca gag ttt ata aga aat tgg gca 304
   Leu Lys Ala Lys Ile Ile Gln Asp Pro Glu Phe Ile Arg Asn Trp Ala
   60 65 70 75
   aat gta gct cat tca tta gga ttt gga tgg tgc ggt gga acg gct aat 352
   Asn Val Ala His Ser Leu Gly Phe Gly Trp Cys Gly Gly Thr Ala Asn
   80 85 90
   cca aac gtt gga caa ggt ttt gaa ttt aaa aga gaa gtt ggg gca ggt 400
   Pro Asn Val Gly Gln Gly Phe Glu Phe Lys Arg Glu Val Gly Ala Gly
   95 100 105
   gga aaa gta tct tat tta tta tct gct aga tac aat cca aat gat cct 448
   Gly Lys Val Ser Tyr Leu Leu Ser Ala Arg Tyr Asn Pro Asn Asp Pro
   110 115 120
   tat gca agt gga tat cgt gca aaa gat aga ctt tct atg aaa ata tca 496
   Tyr Ala Ser Gly Tyr Arg Ala Lys Asp Arg Leu Ser Met Lys Ile Ser
   125 130 135
   aat gtt aga ttt gtt att gat aat gat tct ata aaa tta ggt aca cct 544
   Asn Val Arg Phe Val Ile Asp Asn Asp Ser Ile Lys Leu Gly Thr Pro
   140 145 150 155
   aaa gtg aaa aaa tta gca cct tta aac tct gct agt ttt gat tta ata 592
   Lys Val Lys Lys Leu Ala Pro Leu Asn Ser Ala Ser Phe Asp Leu Ile
   160 165 170
   aat gaa agt aaa act gag tct aaa tta tca aaa aca ttt aat tat aca 640
   Asn Glu Ser Lys Thr Glu Ser Lys Leu Ser Lys Thr Phe Asn Tyr Thr
   175 180 185
   act tct aaa aca gtt tct aaa aca gat aac ttt aaa ttt gga gaa aaa 688
   Thr Ser Lys Thr Val Ser Lys Thr Asp Asn Phe Lys Phe Gly Glu Lys
   190 195 200
   ata gga gta aaa aca tca ttt aaa gta ggt ctt gaa gct ata gct gac 736
   Ile Gly Val Lys Thr Ser Phe Lys Val Gly Leu Glu Ala Ile Ala Asp
   205 210 215
   agt aaa gtt gag aca agc ttt gaa ttt aat gca gaa caa ggt tgg tca 784
   Ser Lys Val Glu Thr Ser Phe Glu Phe Asn Ala Glu Gln Gly Trp Ser
   220 225 230 235
   aat aca aat agt act act gaa act aaa caa gaa agt act aca tat act 832
   Asn Thr Asn Ser Thr Thr Glu Thr Lys Gln Glu Ser Thr Thr Tyr Thr
   240 245 250
   gca aca gtt tct cca caa act aaa aag aga tta ttc cta gat gtg tta 880
   Ala Thr Val Ser Pro Gln Thr Lys Lys Arg Leu Phe Leu Asp Val Leu
   255 260 265
   gga tca caa att gat att cct tat gaa gga aaa ata tat atg gaa tac 928
   Gly Ser Gln Ile Asp Ile Pro Tyr Glu Gly Lys Ile Tyr Met Glu Tyr
   270 275 280
   gac ata gaa tta atg gga ttt tta aga tat aca gga aat gct cgt gaa 976
   Asp Ile Glu Leu Met Gly Phe Leu Arg Tyr Thr Gly Asn Ala Arg Glu
   285 290 295
   gat cat act gaa gat aga cca aca gtt aaa ctt aaa ttt ggt aaa aac 1024
   Asp His Thr Glu Asp Arg Pro Thr Val Lys Leu Lys Phe Gly Lys Asn
   300                 305                 310                 315
   ggt atg agt gct gag gaa cat ctt aaa gat tta tat agt cat aag aat     1072
   Gly Met Ser Ala Glu Glu His Leu Lys Asp Leu Tyr Ser His Lys Asn
                   320                 325                 330
   att aat gga tat tca gaa tgg gat tgg aaa tgg gta gat gag aaa ttt     1120
   Ile Asn Gly Tyr Ser Glu Trp Asp Trp Lys Trp Val Asp Glu Lys Phe
               335                 340                 345
   ggt tat tta ttt aaa aat tca tac gat gct ctt act agt aga aaa tta     1168
   Gly Tyr Leu Phe Lys Asn Ser Tyr Asp Ala Leu Thr Ser Arg Lys Leu
           350                 355                 360
   gga gga ata ata aaa ggc tca ttt act aac att aat gga aca aaa ata     1216
   Gly Gly Ile Ile Lys Gly Ser Phe Thr Asn Ile Asn Gly Thr Lys Ile
       365                 370                 375
   gta att aga gaa ggt aaa gaa att cca ctt cct gat aag aag aga aga     1264
   Val Ile Arg Glu Gly Lys Glu Ile Pro Leu Pro Asp Lys Lys Arg Arg
   380                 385                 390                 395
   gga aaa cgt tca gta gat tct tta gat gct aga tta caa aat gaa ggt     1312
   Gly Lys Arg Ser Val Asp Ser Leu Asp Ala Arg Leu Gln Asn Glu Gly
                   400                 405                 410
   att aga ata gaa aat att gaa aca caa gat gtt cca gga ttt aga cta     1360
   Ile Arg Ile Glu Asn Ile Glu Thr Gln Asp Val Pro Gly Phe Arg Leu
               415                 420                 425
   aat agc ata aca tac aat gat aaa aaa ttg ata tta att aat aat ata     1408
   Asn Ser Ile Thr Tyr Asn Asp Lys Lys Leu Ile Leu Ile Asn Asn Ile
           430                 435                 440
   taa ttataattta ttaaaatatg cttctctata ctttatatta atatttaaag          1461
   tataaaaact aacaaaatct cacttagtag gtagaattgt ataaaaacaa atctacctac   1521
   tattttttta ttatttagtc g                                              1542

<210> 8

<211> 443

<212> PRT

<213> Clostridium septicum

<400> 8

Met Ser Lys Lys Ser Phe Ala Lys Lys Val Ile Cys Thr Ser Met Ile
1               5                   10                  15

Ala Ile Gln Cys Ala Ala Val Val Pro His Val Gln Ala Tyr Ala Leu
            20                  25                  30

Thr Asn Leu Glu Glu Gly Gly Tyr Ala Asn His Asn Asn Ala Ser Ser
        35                  40                  45

Ile Lys Ile Phe Gly Tyr Glu Asp Asn Glu Asp Leu Lys Ala Lys Ile
    50                  55                  60

Ile Gln Asp Pro Glu Phe Ile Arg Asn Trp Ala Asn Val Ala His Ser
65                  70                  75                  80

Leu Gly Phe Gly Trp Cys Gly Gly Thr Ala Asn Pro Asn Val Gly Gln
                85                  90                  95

Gly Phe Glu Phe Lys Arg Glu Val Gly Ala Gly Gly Lys Val Ser Tyr
            100                 105                 110

Leu Leu Ser Ala Arg Tyr Asn Pro Asn Asp Pro Tyr Ala Ser Gly Tyr
        115                 120                 125

Arg Ala Lys Asp Arg Leu Ser Met Lys Ile Ser Asn Val Arg Phe Val
    130                 135                 140

Ile Asp Asn Asp Ser Ile Lys Leu Gly Thr Pro Lys Val Lys Lys Leu
145                 150                 155                 160

Ala Pro Leu Asn Ser Ala Ser Phe Asp Leu Ile Asn Glu Ser Lys Thr
                165                 170                 175

Glu Ser Lys Leu Ser Lys Thr Phe Asn Tyr Thr Thr Ser Lys Thr Val

            180                 185                 190

Ser Lys Thr Asp Asn Phe Lys Phe Gly Glu Lys Ile Gly Val Lys Thr

        195                 200                 205

Ser Phe Lys Val Gly Leu Glu Ala Ile Ala Asp Ser Lys Val Glu Thr

    210                 215                 220

Ser Phe Glu Phe Asn Ala Glu Gln Gly Trp Ser Asn Thr Asn Ser Thr

225                 230                 235                 240

Thr Glu Thr Lys Gln Glu Ser Thr Thr Tyr Thr Ala Thr Val Ser Pro

                245                 250                 255

Gln Thr Lys Lys Arg Leu Phe Leu Asp Val Leu Gly Ser Gln Ile Asp

            260                 265                 270

Ile Pro Tyr Glu Gly Lys Ile Tyr Met Glu Tyr Asp Ile Glu Leu Met

        275                 280                 285

Gly Phe Leu Arg Tyr Thr Gly Asn Ala Arg Glu Asp His Thr Glu Asp

    290                 295                 300

Arg Pro Thr Val Lys Leu Lys Phe Gly Lys Asn Gly Met Ser Ala Glu

305                 310                 315                 320

Glu His Leu Lys Asp Leu Tyr Ser His Lys Asn Ile Asn Gly Tyr Ser

                325                 330                 335

Glu Trp Asp Trp Lys Trp Val Asp Glu Lys Phe Gly Tyr Leu Phe Lys

            340                 345                 350

Asn Ser Tyr Asp Ala Leu Thr Ser Arg Lys Leu Gly Gly Ile Ile Lys

        355                 360                 365

Gly Ser Phe Thr Asn Ile Asn Gly Thr Lys Ile Val Ile Arg Glu Gly

    370                 375                 380

Lys Glu Ile Pro Leu Pro Asp Lys Lys Arg Arg Gly Lys Arg Ser Val

385                 390                 395                 400

Asp Ser Leu Asp Ala Arg Leu Gln Asn Glu Gly Ile Arg Ile Glu Asn

405                 410                 415

Ile Glu Thr Gln Asp Val Pro Gly Phe Arg Leu Asn Ser Ile Thr Tyr

            420                 425                 430

Asn Asp Lys Lys Leu Ile Leu Ile Asn Asn Ile

        435                 440

<210>  9

<211>  20

<212>  DNA

<213>  Artificial

<220>

<223>  Primer

<400>  9

aggtcgtgta ctgtcagtca                                                  20

<210>  10

<211>  20

<212>  DNA

<213>  Artificial

<220>

<223>  Primer

<400>  10

acgtggtgaa ctgccagtga                                                  20

## Description

### FIELD

This application relates to novel proteins that include aerolysin (or aerolysin homologs) and a lung cancer-specific binding agent, nucleic acid molecules that encode such proteins, and methods of their use to treat lung cancer.

### BACKGROUND

Non-small cell lung cancer accounts for almost 80% of all lung cancers. This group of cancers includes adenocarcinomas, which account for approximately 40% of all cases of non-small cell lung cancer; squamous cell carcinomas, which include approximately 30% of all cases of non-small cell lung cancer; and large cell carcinomas, which account for about 10-15% of all non-small cell lung cancers.

Bacterial toxins, such as aerolysin produced by *Aeromonas hydrophilia* and α-hemolysin produced by *Staph aureus,* are beta-sheet proteins that oligomerize and insert into the plasma membrane to produce pores that lead to rapid cytolytic cell death. Pore formation physically disrupts the cell membranes, and results in death of cells in all phases of the cell cycle, including non-proliferating cells (GO arrested). However, these toxins, including aerolysin, kill cells indiscriminately. Therefore, compositions and methods for utilizing these toxins to kill tumor cells are needed

### SUMMARY

Disclosed herein are modified aerolysin molecules and methods of their use for treatment of localized and metastatic lung cancers, such as non-small cell lung cancers.

In one example, a modified aerolysin molecule includes a lung cancer specific binding agent, such as AFAI. In some examples, a modified aerolysin molecule includes an aerolysin homolog, such as *Clostridium septicum* alpha toxin, and a lung cancer specific binding agent, such as AFAI. Pharmaceutical compositions that include the disclosed modified aerolysin molecules are also provided.

Methods are disclosed for treatment of localized and metastatic lung cancers using the disclosed modified aerolysin molecules. In addition, methods are disclosed for stimulating a subject's immune system to enhance treatment of localized and metastatic lung cancer. Administration of the disclosed molecules to a subject having lung cancer results in targeting of the toxin specifically to lung cancer cells, activation of the toxin, and lysis of the lung cancer cells.

### BRIEF DESCRIPTION OF THE FIGURES

**FIG. 1** is a schematic drawing showing the cloning site region of pSJF2 (a pUC-based vector). Since sdAb fusion is cloned using HindIII, the construct does not have a C-terminal c-myc-his5.
**FIG. 2** is a bar graph showing the results of an LDH activity assay showing the cytotoxicity of AFAI-aerolysin towards A549 lung cancer cells.

### SEQUENCE LISTING

The nucleic and amino acid sequences listed in the accompanying sequence listing are shown using standard letter abbreviations for nucleotide bases, and three letter code for amino acids. Only one strand of each nucleic acid sequence is shown, but the complementary strand is understood as included by any reference to the displayed strand.

SEQ ID NOS: 1-4 show nucleic acid primers used to generate the AFAI-aerolysin hybrid sequence.

SEQ ID NOS: 5 and 6 show an AFAI-aerolysin hybrid cDNA and protein sequence, respectively. The aerolysin sequence starts at amino acid 136 of SEQ ID NO: 6.

SEQ ID NOS: 7 and 8 show an alpha toxin cDNA and protein sequence, respectively (see Genbank Accession No. S75954).

### DETAILED DESCRIPTION OF SEVERAL EMBODIMENTS

### Abbreviations and Terms

The following explanations of terms and methods are provided to better describe the present disclosure and to guide those of ordinary skill in the art in the practice of the present disclosure. As used herein and in the appended claims, the singular forms "a" or "an" or "the" include plural references unless the context clearly dictates otherwise. For example, reference to "a modified aerolysin molecule" includes a plurality of such molecules and reference to "the antibody" includes reference to one or more antibodies and equivalents thereof known to those skilled in the art, and so forth. The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements.

Unless explained otherwise, all technical and scientific terms used herein have the same meaning as commonly understood to one of ordinary skill in the art to which this disclosure belongs.

**Aerolysin**: A channel-forming toxin that in its native form is produced as an inactive protoxin called proaerolysin (PA). Aerolysin includes many discrete functionalities such as a binding domain, a toxin domain (approximately amino acids 136-482 of SEQ ID NO: 6), and a C-terminal inhibitory peptide domain (approximately amino acids 483-526 of SEQ ID NO: 6) that contains a protease activation site (amino acids 483-488 of SEQ ID NO: 6).

The binding domain recognizes and binds to glycophosphatidylinositol (GPI) membrane anchors, such as are found in Thy-1 on T lymphocytes, human placental alkaline phosphatase and prostate specific membrane antigen. Most mammalian cells express GPI anchored proteins on their surfaces. The activation or proteolysis site within proaerolysin is a six amino acid sequence that is recognized as a proteolytic substrate by the furin family of proteases. When furin hydrolyses the activation site, a C-terminal inhibitory segment is released, resulting in active aerolysin. Aerolysin binds to GPI-anchored proteins on the cell membrane and forms a heptamer that inserts into the membrane producing well-defined channels of ~ 17 Å. Channel formation leads to rapid cell death via necrosis. Wild-type aerolysin is toxic to mammalian cells, including erythrocytes, for example at 1 nanomolar or less.

Includes any aerolysin gene, cDNA, RNA, or protein from any *Aeromonas* organism. This description includes aerolysin allelic variants, as well as any variant, fragment, or fusion sequence that retains the ability to bind GPI-anchored proteins on the cell membrane and form channels in the cell membrane, which leads to cell death.

An example of an aerolysin nucleic acid sequence includes the sequence provided in GenBank Accession No. M16495. In particular examples, an aerolysin nucleic acid sequence includes the sequence shown in nucleotides 406-1578 of SEQ ID NO: 5, or fragments, variants, or fusions thereof that retain the ability to encode a protein having modified aerolysin activity. An example of an aerolysin protein sequence includes the sequence provided in GenBank Accession No. AAA21938, or fragments, fusions, or variants thereof that retain modified aerolysin activity. In another example, an aerolysin protein includes the amino acid sequence shown in amino acids 136-526 of SEQ ID NO: 6, or fragments, fusions, or variants thereof that retain modified aerolysin activity.

In one example, aerolysin includes homologs of aerolysin, such as the pore-forming alpha toxin produced by *Clostridium septicum.*

**Agent**: Any protein, nucleic acid molecule, compound, small molecule, organic compound, inorganic compound, or other molecule of interest.

**Alpha toxin:** A pore-forming toxin produced by *Clostridium septicum* that belongs to the aerolysin-like family of pore-forming toxins. Alpha toxin is secreted as an inactive 46,450-Da protoxin, which is activated by proteolytic cleavage near the C terminus, which eventually causes the release of a 45-amino-acid fragment. Proteoytic activation and loss of the propeptide allow alpha-toxin to oligomerize and form pores on the plasma membrane, which results in colloidal-osmotic lysis.

Includes any alpha toxin gene, cDNA, RNA, or protein from any *Clostridium* organism. An example of an alpha toxin nucleic acid sequence includes the sequence provided in GenBank Accession No. S75954 (SEQ ID NO: 7) or fragments, variants, or fusions thereof that retain the ability to encode a peptide or protein having alpha toxin activity. An example of an alpha toxin protein sequence includes the sequence provided in GenBank Accession No. AAB32892 (SEQ ID NO: 8), or fragments, fusions, or variants thereof that retain alpha toxin activity.

**Antibody**: A molecule including an antigen-binding site which specifically binds (immunoreacts with) an antigen. Examples include polyclonal antibodies, monoclonal antibodies, humanized monoclonal antibodies, or immunologically effective portions thereof. In one example, an antibody includes camelized antibodies or llama antibodies (for example see Tanha et al., J. Biol. Chem. 276:24774-80, 2001).

Includes immunoglobulin molecules and immunologically active portions thereof. Naturally occurring antibodies (for example IgG) include four polypeptide chains, two heavy (H) chains and two light (L) chains inter-connected by disulfide bonds. However, the antigen-binding function of an antibody can be performed by fragments of a naturally occurring antibody. Immunologically effective portions of monoclonal antibodies include, but are not limited to: Fab, Fab', F(ab')₂, Fabc and Fv portions (for a review, see Better and Horowitz, Methods. Enzymol. 178:476-96, 1989). Other examples of antigen-binding fragments include, but are not limited to: (i) a Fab fragment consisting of the VL, VH, CL and CHl domains; (ii) an Fd fragment consisting of the VH and CHl domains; (iii) an Fv fragment consisting of the VL and VH domains of a single arm of an antibody, (iv) a dAb fragment which consists of a VH domain; (v) an isolated complimentarity determining region (CDR); and (vi) an F(ab')₂ fragment, a bivalent fragment comprising two Fab fragments linked by a disulfide bridge at the hinge region. Furthermore, although the two domains of the Fv fragment are coded for by separate genes, a synthetic linker can be made that enables them to be made as a single protein chain (known as single chain Fv, scFv) (Bird et al. Science 242:423-6, 1988; and Huston et al., Proc. Natl. Acad Sci. 85:5879-83, 1988) by recombinant methods. Such single chain antibodies are also included.

In one example, antibody fragments are capable of crosslinking their target antigen, for example bivalent fragments such as F(ab')2 fragments. Alternatively, an antibody fragment which does not itself crosslink its target antigen (such as a Fab fragment) can be used in conjunction with a secondary antibody which serves to crosslink the antibody fragment, thereby crosslinking the target antigen. Antibodies can be fragmented using conventional techniques and the fragments screened for utility in the same manner as described for whole antibodies. An antibody is further intended to include bispecific and chimeric molecules that specifically bind the target antigen.

"Specifically binds" refers to the ability of a particular agent (a "specific binding agent") to specifically react with a particular analyte, for example to specifically immunoreact with an antibody, or to specifically bind to a particular peptide sequence. The binding is a non-random binding reaction, for example between an antibody molecule and an antigenic determinant. Binding specificity of an antibody is typically determined from the reference point of the ability of the antibody to differentially bind the specific antigen and an unrelated antigen, and therefore distinguish between two different antigens, particularly where the two antigens have unique epitopes. An antibody that specifically binds to a particular epitope is referred to as a "specific antibody". Specific binding agents include any agent that binds substantially only to a defined target. The determination that an antibody specifically binds to a particular polypeptide is made by any one of a number of standard immunoassay methods; for instance, Western blotting.

**Cancer**: Malignant neoplasm that has undergone characteristic anaplasia with loss of differentiation, increase rate of growth, invasion of surrounding tissue, and is capable of metastasis.

**cDNA (complementary DNA):** A piece of DNA lacking internal, non-coding segments (introns) and regulatory sequences which determine transcription. cDNA can be synthesized in the laboratory by reverse transcription from messenger RNA extracted from cells.

**Chemical synthesis:** An artificial means by which one can make a protein or peptide. A synthetic protein or peptide is one made by such artificial means.

**Chemotherapy**: In cancer treatment, chemotherapy refers to the administration of one or a combination of compounds to kill or slow the reproduction of rapidly multiplying cells. Chemotherapuetic agents include but are not limited to: 5-fluorouracil (5-FU), azathioprine, cyclophosphamide, antimetabolites (such as Fludarabine), antineoplastics (such as Etoposide, Doxorubicin, methotrexate, and Vincristine), carboplatin, cis-platinum and the taxanes, such as taxol and taxotere. Such agents can be co-administered with the disclosed modified aerolysin molecules to a subject. Chemotherapeutic agents can also be administered prior to or subsequent to administration of the disclosed modified aerolysin molecules to a subject. In one example, chemotherapeutic agents are co-administered with radiation therapy, along with the disclosed modified aerolysin molecules, such as AFAI-aerolysin, for treatment of a localized lung carcinoma.

**Conservative substitution:** A substitution of an amino acid residue for another amino acid residue having similar biochemical properties. Typically, conservative substitutions have little to no impact on the biological activity of a resulting polypeptide. In a particular example, a conservative substitution is an amino acid substitution in a peptide that does not substantially affect the biological function of the peptide. A peptide can include one or more amino acid substitutions, for example 2-10 conservative substitutions, 2-5 conservative substitutions, 4-9 conservative substitutions, such as 2, 5 or 10 conservative substitutions.

For example, ideally, a modified aerolysin peptide including one or more conservative substitutions retains modified aerolysin activity. A polypeptide can be produced to contain one or more conservative substitutions by manipulating the nucleotide sequence that encodes that polypeptide using, for example, standard procedures such as site-directed mutagenesis or PCR. In one example, such variants can be readily selected for additional testing by performing an assay (such as those described in Examples 2 and 4-7) to determine if the variant retains modified aerolysin activity.

A polypeptide can be produced to contain one or more conservative substitutions by manipulating the nucleotide sequence that encodes that polypeptide using, for example, standard procedures such as site-directed mutagenesis or PCR. Alternatively, a polypeptide can be produced to contain one or more conservative substitutions by using standard peptide synthesis methods. An alanine scan can be used to identify which amino acid residues in a protein can tolerate an amino acid substitution. In one example, the biological activity of the protein is not decreased by more than 25%, for example not more than 20%, for example not more than 10%, when an alanine, or other conservative amino acid (such as those listed below), is substituted for one or more native amino acids.

Substitutional variants are those in which at least one residue in the amino acid sequence has been removed and a different residue inserted in its place. Examples of amino acids which can be substituted for an original amino acid in a protein and which are regarded as conservative substitutions include, but are not limited to: Ser for Ala; Lys for Arg; Gln or His for Asn; Glu for Asp; Ser for Cys; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

Permissive substitutions are non-conservative amino acid substitutions, but also do not significantly alter modified aerolysin activity.

Further information about conservative substitutions can be found in, among other locations in, Ben-Bassat et al., (J. Bacteriol. 169:751-7, 1987), O'Regan et al., (Gene 77:237-51, 1989), Sahin-Toth et al., (Protein Sci. 3:240-7, 1994), Hochuli et al., (Bio/Technology 6:1321-5, 1988) and in standard textbooks of genetics and molecular biology.

**Deletion**: The removal of a sequence of a nucleic acid, for example DNA, the regions on either side being joined together.

**DNA (Deoxyribonucleic acid):** A long chain polymer which includes the genetic material of most living organisms (some viruses have genes comprising ribonucleic acid, RNA). The repeating units in DNA polymers are four different nucleotides, each of which comprises one of the four bases, adenine, guanine, cytosine and thymine bound to a deoxyribose sugar to which a phosphate group is attached. Triplets of nucleotides, referred to as codons, in DNA molecules code for amino acid in a polypeptide. The term codon is also used for the corresponding (and complementary) sequences of three nucleotides in the mRNA into which the DNA sequence is transcribed.

**Enhance:** To improve the quality, amount, or strength of something. In one example, a therapy enhances the ability of a subject to reduce tumors, such as a lung carcinoma, if the subject is more effective at fighting tumors. In another example, a therapy enhances the ability of an agent to reduce tumors, such as a lung carcinoma, in a subject if the agent is more effective at reducing tumors. Such enhancement can be measured using the methods disclosed herein, for example determining the decrease in tumor volume (see Example 5).

**Exogenous:** The term "exogenous" as used herein with reference to nucleic acid and a particular cell refers to any nucleic acid that does not originate from that particular cell as found in nature. Thus, a non-naturally-occurring nucleic acid is considered to be exogenous to a cell once introduced into the cell. A nucleic acid that is naturally-occurring also can be exogenous to a particular cell. For example, an entire chromosome isolated from a cell of person X is an exogenous nucleic acid with respect to a cell of person Y once that chromosome is introduced into Y's cell.

**Hybridization**: To form base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the Na+ concentration) of the hybridization buffer will determine the stringency of hybridization. Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11). The following is an exemplary set of hybridization conditions and is not limiting:

| Very High Stringency (detects sequences that share 90% identity) | |
|---|---|
| Hybridization: | 5x SSC at 65°C for 16 hours |
| Wash twice: | 2x SSC at room temperature (RT) for 15 minutes each |
| Wash twice: | 0.5x SSC at 65°C for 20 minutes each |

| High Stringency (detects sequences that share 80% identity or greater) | |
|---|---|
| Hybridization: | 5x-6x SSC at 65°C-70°C for 16-20 hours |
| Wash twice: | 2x SSC at RT for 5-20 minutes each |
| Wash twice: | 1x SSC at 55°C-70°C for 30 minutes each |

| Low Stringency (detects sequences that share greater than 50% identity) | |
|---|---|
| Hybridization: | 6x SSC at RT to 55°C for 16-20 hours |
| Wash at least twice: | 2x-3x SSC at RT to 55°C for 20-30 minutes each. |

**Immobilized**: Bound to a surface, such as a solid surface. A solid surface can be polymeric, such as polystyrene or polypropylene. In one example, the solid surface is in the form of a bead. In another example, the surface includes a modified aerolysin protein, such as AFAI-aerolysin. Ideally, modified aerolysin toxin is liberated from the bead once the bead reaches the lung cancer cell target. Methods of immobilizing peptides on a solid surface can be found in WO 94/29436, and U.S. Patent No. 5,858,358.

**Insertion**: The addition of one or more nucleotides to a nucleic acid sequence, or the addition of one or more amino acids to a protein sequence.

**Isolated**: An "isolated" biological component (such as a nucleic acid molecule or protein) has been substantially separated or purified away from other biological components in the cell of the organism in which the component naturally occurs (such as other chromosomal and extrachromosomal DNA and RNA). Nucleic acids and proteins that have been "isolated" include nucleic acids and proteins purified by standard purification methods. The term also embraces nucleic acids and proteins prepared by recombinant expression in a host cell as well as chemically synthesized nucleic acids and proteins.

**Lung cancer specific binding agent:** Any agent that specifically recognizes and binds to a lung cancer cell, and ideally does not bind to other cells, such as non-malignant lung cells. Includes antibodies and other agents, such as peptides or drugs, which bind substantially to only a lung cancer cell, such as a protein on the cell. In particular examples, lung cancer-specific binding agents bind to metastatic neoplasia of lung origin found elsewhere in the body. Particular examples include AFAI (amino acids 1-135 of SEQ ID NO: 6 as well as variants, fragments, and fusions thereof that retain the ability to specifically bind lung cancer cells), as well as antibodies that recognize carcino embryonic antigen CEA (for example catalog number ab10036 from Abcam Inc., Cambridge, MA), thyroid transcription factor 1 (TTF1) (for example catalog number ab869 from Abcam Inc., Cambridge, MA), and cytokeratin 7 (for example catalog number RDI-PRO61025 from Research Diagnostics Inc., Flanders NJ). Those skilled in the art are capable of identifying other lung cancer-specific binding agents.

**Lung tumor cell volume:** The amount of 3-dimensional space occupied by a lung tumor, such as a tumor including lung cancer cells.

**Malignant**: Cells that have the properties of anaplasia invasion and metastasis.

**Mammal**: This term includes both human and non-human mammals. Examples of mammals include, but are not limited to: humans, pigs, cows, goats, cats, dogs, rabbits and mice.

**Modified aerolysin proteins:** Proteins that include an aerolysin sequence linked to one or more lung-cancer-specific binding agents, such as AFAI. Such proteins permit the targeting of aerolysin to lung cancer cells. In particular examples, such proteins include a lung-cancer-specific antibody attached to the N-terminus of aerolysin (for example see SEQ ID NOS: 5 and 6), the C-terminus of aerolysin, or cross-linked to any reside of aerolysin.

**Modified aerolysin activity:** The ability of a modified aerolysin molecule (such as modified aerolysin proteins and nucleic acids encoded thereby) to be targeted to a lung cancer cell, such as a non-small cell lung cancer cell, and lyse the cancer cell.

In one example, modified aerolysin activity includes the ability of a modified aerolysin protein, when contacted with a lung cancer cell, to increase lysis and death of the cell, for example by at least 10%, at least 25%, at least 50%, at least 100%, at least 200% or even at least 500%, when compared to an amount of lysis of a non-lung cancer cell, or compared to an amount of lung-cancer cell lysis in the absence of the therapeutic agent.

In one example, modified aerolysin activity includes the ability of a modified aerolysin protein, when contacted with a tumor, to decrease tumor cell volume, such as a lung tumor volume, for example by at least 10%, at least 20%, at least 30%, at least 40%, at least 50%, at least 60%, at least 70%, at least 75%, at least 80%, at least 85%, at least 90%, at least 95%, at least 98%, or even at least 100% (complete elimination of the tumor), when compared to a tumor cell volume in the absence of the therapeutic agent.

Assays which can be used to determine if an agent has modified aerolysin activity are described herein, for example in Examples 2 and 4-7. For example, a modified aerolysin peptide can be assessed for its ability to specifically lyse lung cancer cells. Functional protein activity could be detected by the preferential lysis of lung cancer cells versus non-lung cancer cells, decreasing lung tumor volume, and having a decreased toxicity when compared to aerolysin alone.

Similar assays can be used to determine if any modified aerolysin agent disclosed herein can decrease tumor volume (such as a lung tumor) and specifically lyse lung cancer cells. For example, the modified aerolysin peptide shown in SEQ ID NO: 6 is expected to decrease lung tumor volume. Any of these assays can be modified by using *in vivo* expression of a modified aerolysin nucleic acid molecule (including variants, fusions, and fragments thereof), instead of administration of purified proteins.

**Neoplasm:** Abnormal growth of cells.

**Normal Cell:** Non-tumor cell, non-malignant, uninfected cell.

**Oligonucleotide:** A linear polynucleotide sequence of up to about 200 nucleotide bases in length, for example a polynucleotide (such as DNA or RNA) which is at least about 6 nucleotides, for example at least 15, 50, 100 or 200 nucleotides long.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

**ORF (open reading frame):** A series of nucleotide triplets (codons) coding for amino acids without any termination codons. These sequences are usually translatable into a peptide.

**Pharmaceutical agent:** A chemical compound or composition capable of inducing a desired therapeutic or prophylactic effect when administered to a subject, alone or in combination with another therapeutic agent(s) or pharmaceutically acceptable carriers. In a particular example, a pharmaceutical agent decreases lung tumor cell volume, such as a tumor in a subject.

**Polynucleotide:** A linear nucleic acid sequence of any length. Therefore, a polynucleotide includes molecules which are at least 15, 50, 100, 200, 400, 500, 1000, 1100, or 1200 (oligonucleotides) and also nucleotides as long as a full-length cDNA or chromosome.

**Preventing or treating a disease:** "Preventing" a disease refers to inhibiting the full development of a disease, for example preventing metastasis of a lung tumor. "Treatment" refers to a therapeutic intervention that ameliorates a sign or symptom of a disease or pathological condition related to lung cancer, such as reducing lung tumor volume. Treatment can involve slowing the growth or metastasis of a lung tumor, and can also include halting or reversing the growth or metastasis of a lung tumor permanently. However, treatment does not require complete inhibition of tumor cell growth or a 100% reduction in tumor cell volume.

**Promoter:** An array of nucleic acid control sequences which direct transcription of a nucleic acid. A promoter includes necessary nucleic acid sequences near the start site of transcription, such as, in the case of a polymerase II type promoter, a TATA element. A promoter also optionally includes distal enhancer or repressor elements that can be located as much as several thousand base pairs from the start site of transcription.

**Purified:** The term "purified" does not require absolute purity; rather, it is intended as a relative term. Thus, for example, a substantially purified protein or nucleic acid preparation (such as the modified aerolysin toxins disclosed herein) is one in which the protein or nucleic acid referred to is more pure than the protein in its natural environment within a cell or within a production reaction chamber (as appropriate). For example, a preparation of a modified aerolysin protein is purified if the protein represents at least 50%, for example at least 70%, of the total protein content of the preparation. Methods for purification of proteins and nucleic acids are well known in the art. Examples of methods that can be used to purify a protein, such as a modified aerolysin include, but are not limited to the methods disclosed in Sambrook *et al.* (*Molecular Cloning: A Laboratory Manual,* Cold Spring Harbor, New York, 1989, Ch. 17).

**Recombinant:** A recombinant nucleic acid is one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, for example by genetic engineering techniques. A recombinant protein is one that results from expressing a recombinant nucleic acid encoding the protein.

**Sample:** Biological samples containing genomic DNA, cDNA, RNA, or protein obtained from the cells of a subject, such as those present in peripheral blood, urine, saliva, semen, tissue biopsy, surgical specimen, fine needle aspriates, amniocentesis samples and autopsy material. In one example, a sample includes lung cancer cells obtained from a subject.

**Sequence identity:** The identity/similarity between two or more nucleic acid sequences, or two or more amino acid sequences, is expressed in terms of the identity or similarity between the sequences. Sequence identity can be measured in terms of percentage identity; the higher the percentage, the more identical the sequences are. Sequence similarity can be measured in terms of percentage similarity (which takes into account conservative amino acid substitutions); the higher the percentage, the more similar the sequences are. Homologs or orthologs of nucleic acid or amino acid sequences possess a relatively high degree of sequence identity/similarity when aligned using standard methods. This homology is more significant when the orthologous proteins or cDNAs are derived from species that are more closely related (such as human and mouse sequences), compared to species more distantly related (such as human and *C. elegans* sequences).

Methods of alignment of sequences for comparison are well known in the art. Various programs and alignment algorithms are described in: Smith & Waterman, Adv. Appl. Math. 2:482, 1981; Needleman & Wunsch, J. Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc. Acids Res. 16:10881-90, 1988; Huang et al. Computer Appls. in the Biosciences 8, 155-65, 1992; and Pearson et al., Meth. Mol. Bio. 24:307-31, 1994. Altschul et al., J. Mol. Biol. 215:403-10, 1990, presents a detailed consideration of sequence alignment methods and homology calculations.

The NCBI Basic Local Alignment Search Tool (BLAST) (Altschul et al., J. Mol. Biol. 215:403-10, 1990) is available from several sources, including the National Center for Biological Information (NCBI, National Library of Medicine, Building 38A, Room 8N805, Bethesda, MD 20894) and on the Internet, for use in connection with the sequence analysis programs blastp, blastn, blastx, tblastn and tblastx. Additional information can be found at the NCBI web site.

BLASTN is used to compare nucleic acid sequences, while BLASTP is used to compare amino acid sequences. To compare two nucleic acid sequences, the options can be set as follows: -i is set to a file containing the first nucleic acid sequence to be compared (such as C:\seq1.txt); -j is set to a file containing the second nucleic acid sequence to be compared (such as C:\seq2.txt); -p is set to blastn; -o is set to any desired file name (such as C:\output.txt); -q is set to -1; -r is set to 2; and all other options are left at their default setting. For example, the following command can be used to generate an output file containing a comparison between two sequences: C:\Bl2seq -i c:\seq1.txt -j c:\seq2.txt -p blastn -o c:\output.txt -q -1 -r 2.

To compare two amino acid sequences, the options of B12seq can be set as follows: -i is set to a file containing the first amino acid sequence to be compared (such as C:\seq1.txt); -j is set to a file containing the second amino acid sequence to be compared (such as C:\seq2.txt); -p is set to blastp; -o is set to any desired file name (such as C:\output.txt); and all other options are left at their default setting. For example, the following command can be used to generate an output file containing a comparison between two amino acid sequences: C:\B12seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences.

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (such as 100 consecutive nucleotides or amino acid residues from a sequence set forth in an identified sequence), followed by multiplying the resulting value by 100. For example, a nucleic acid sequence that has 1166 matches when aligned with a test sequence having 1154 nucleotides is 75.0 percent identical to the test sequence (1166÷1554*100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer. In another example, a target sequence containing a 20-nucleotide region that aligns with 20 consecutive nucleotides from an identified sequence as follows contains a region that shares 75 percent sequence identity to that identified sequence(15÷20*100=75).

1                    20
Target Sequence:        AGGTCGTGTACTGTCAGTCA
                        | || ||| |||| |||| |
Identified Sequence:    ACGTGGTGAACTGCCAGTGA

For comparisons of amino acid sequences of greater than about 30 amino acids, the Blast 2 sequences function is employed using the default BLOSUM62 matrix set to default parameters, (gap existence cost of 11, and a per residue gap cost of 1). Homologs are typically characterized by possession of at least 70% sequence identity counted over the full-length alignment with an amino acid sequence using the NCBI Basic Blast 2.0, gapped blastp with databases such as the nr or swissprot database. Queries searched with the blastn program are filtered with DUST (Hancock and Armstrong, 1994, Comput. Appl. Biosci. 10:67-70). Other programs use SEG. In addition, a manual alignment can be performed. Proteins with even greater similarity will show increasing percentage identities when assessed by this method, such as at least 75%, 80%, 85%, 90%, 95%, or 99% sequence identity.

When aligning short peptides (fewer than around 30 amino acids), the alignment should be performed using the Blast 2 sequences function, employing the PAM30 matrix set to default parameters (open gap 9, extension gap 1 penalties). Proteins with even greater similarity to the reference sequence will show increasing percentage identities when assessed by this method, such as at least 60%, 70%, 75%, 80%, 85%, 90%, 95%, 98%, or 99% sequence identity. When less than the entire sequence is being compared for sequence identity, homologs will typically possess at least 75% sequence identity over short windows of 10-20 amino acids, and can possess sequence identities of at least 85%, 90%, 95% or 98% depending on their identity to the reference sequence. Methods for determining sequence identity over such short windows are described at the NCBI web site.

One indication that two nucleic acid molecules are closely related is that the two molecules hybridize to each other under stringent conditions. Stringent conditions are sequence-dependent and are different under different environmental parameters. Nucleic acid molecules that hybridize under stringent conditions to a modified aerolysin nucleic acid sequence typically hybridize to a probe based on either an entire modified aerolysin nucleic acid sequence or selected portions of the sequence, respectively, under conditions described above.

Nucleic acid sequences that do not show a high degree of identity may nevertheless encode identical or similar (conserved) amino acid sequences, due to the degeneracy of the genetic code. Changes in a nucleic acid sequence can be made using this degeneracy to produce multiple nucleic acid molecules that all encode substantially the same protein. Such homologous nucleic acid sequences can, for example, possess at least 60%, 70%, 80%, 90%, 95%, 98%, or 99% sequence identity determined by this method.

One of skill in the art will appreciate that these sequence identity ranges are provided for guidance only; it is possible that strongly significant homologs could be obtained that fall outside the ranges provided.

An alternative (and not necessarily cumulative) indication that two nucleic acid sequences are substantially identical is that the polypeptide which the first nucleic acid encodes is immunologically cross reactive with the polypeptide encoded by the second nucleic acid.

**Subject:** Living multicellular vertebrate organisms, a category which includes both human and veterinary subjects that are in need of the desired biological effect. Examples include, but are not limited to: humans, apes, dogs, cats, mice, rats, rabbits, horses, pigs, and cows.

**Therapeutically Effective Amount:** An amount of a pharmaceutical preparation that alone, or together with an additional therapeutic agent(s) (for example a chemotherapeutic agent), induces the desired response. The preparations disclosed herein are administered in therapeutically effective amounts.

In one example, a desired response is to decrease the size, such as the volume of a lung tumor or metastasis in a subject to whom the therapy is administered. The volume of the tumor does not need to be completely eliminated for the pharmaceutical preparation to be effective. For example, a pharmaceutical preparation can decrease the volume of a lung tumor or lung metastasis by a desired amount, for example by at least 20%, at least 50%, at least 60%, at least 70%, at least 80%, at least 90%, at least 95%, at least 98%, or even at least 100% (elimination of the tumor), as compared to a volume in the absence of the pharmaceutical preparation.

In particular examples, it is an amount of a modified aerolysin molecule effective to decrease an number of lung cancer cells, such as in a subject to whom it is administered, for example a subject having one or more lung carcinomas. The cancer cells do not need to be completely eliminated for the pharmaceutical preparation to be effective. For example, a pharmaceutical preparation can decrease the number of lung cells by a desired amount, for example by at least 20%, at least 50%, at least 60%, at least 70%, at least 80%, at least 90%, at least 95%, at least 98%, or even at least 100% (elimination of detectable cancer cells), as compared to the number of cancer cells in the absence of the pharmaceutical preparation.

In other examples, it is an amount of a modified aerolysin molecule, a number of lung cancer cells lysed by a modified aerolysin molecule, or both, effective to decrease the metastasis of a lung carcinoma. The metastases resulting from a lung cancer do not need to be completely eliminated for the pharmaceutical preparation to be effective. For example, a pharmaceutical preparation can reduce the number or volume of a metastasis by a desired amount, for example by at least 20%, at least 50%, at least 60%, at least 70%, at least 80%, at least 90%, at least 95%, at least 98%, or even at least 100% (elimination of the tumor), as compared to a number or volume of a metastasis in the absence of the pharmaceutical preparation.

A therapeutically effective amount of modified aerolysin, or lung cancer cells lysed by such a molecule, can be administered in a single dose, or in several doses, for example daily, during a course of treatment. However, the therapeutically effective amount can depend on the subject being treated, the severity and type of the condition being treated, and the manner of administration. For example, a therapeutically effective amount of modified aerolysin can vary from about 1 µg -10 mg per 70 kg body weight if administered iv and about 10 µg -100 mg per 70 kg body weight if administered intratumorally. In addition, a therapeutically effective amount of lung cancer cells lysed by aerolysin can vary from about 10⁶ to 10⁸ cells. Effective amounts also can be determined through various *in vitro, in vivo* or *in situ* assays, including the assays described herein (for example, see Examples 2, and 4-7).

**Transformed:** A transformed cell is a cell into which has been introduced a nucleic acid molecule by molecular biology techniques. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Transgene:** An exogenous nucleic acid sequence supplied by a vector. In one example, a transgene includes any modified aerolysin sequence, such as SEQ ID NO: 5, as well as or variants, fragments, or fusions thereof that retain modified aerolysin activity.

**Transgenic Cell:** Transformed cells which contain foreign, non-native DNA.

**Transgenic mammal:** Transformed mammals that contain foreign, non-native DNA. In one example, the non-native DNA is a modified aerolysin, such as SEQ ID NO: 6, or a nucleic acid sequence which encodes for such a protein (such as SEQ ID NO: 5).

**Tumor:** A neoplasm. Includes solid and hematological (or liquid) tumors. Examples of solid tumors, such as sarcomas and carcinomas, include, but are not limited to: fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, and CNS tumors (such as a glioma, astrocytoma, medulloblastoma, craniopharyogioma, ependymoma, pinealoma, hemangioblastoma, acoustic neuroma, oligodendroglioma, menangioma, melanoma, neuroblastoma and retinoblastoma).

Particular examples of lung cancers include, but are not limited to, non-small cell lung cancer such as adenocarcinomas, squamous cell carcinomas, and large cell carcinomas.

**Variants, fragments or fusions:** The disclosed modified aerolysin nucleic acid sequences, such as SEQ ID NO: 5, and the proteins encoded thereby, include variants, fragments, and fusions thereof that retain modified aerolysin biological activity (such as targeting and lysing lung cancer cells). DNA sequences which encode for a protein or fusion thereof, or a fragment or variant of thereof can be engineered to allow the protein to be expressed in eukaryotic or prokaryotic cells, such as mammalian cells, bacterial cells, insect cells, and plant cells. To obtain expression, the DNA sequence can be altered and operably linked to other regulatory sequences. The final product, which contains the regulatory sequences and the therapeutic protein, is referred to as a vector. This vector can be introduced into the desired cell. Once inside the cell the vector allows the protein to be produced.

One of ordinary skill in the art will appreciate that the DNA can be altered in numerous ways without affecting the biological activity of the encoded protein. For example, PCR can be used to produce variations in the DNA sequence that encodes a protein. Such variants can be variants optimized for codon preference in a host cell used to express the protein, or other sequence changes that facilitate expression.

**Vector:** A nucleic acid molecule as introduced into a host cell, thereby producing a transformed host cell. A vector can include nucleic acid sequences that permit it to replicate in the host cell, such as an origin of replication. A vector can also include one or more selectable marker genes and other genetic elements. An insertional vector is capable of inserting itself into a host nucleic acid.

Additional terms commonly used in molecular genetics can be found in Benjamin Lewin, Genes V published by Oxford University Press, 1994 (ISBN 0-19-854287-9); Kendrew et al (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

### Modified Aerolysin Molecules

Herein disclosed are modified aerolysin and aerolysin homologs (such as *Clostridium septicum* alpha toxin) that can be targeted to lung cancer cells by conjugating such proteins to a lung-specific binding agent. One advantage of the disclosed proteins for treatment of localized and metastatic lung cancer is that it combines a proliferation independent therapy with lung cancer-specific drug delivery, resulting in minimal side effects to patients. One skilled in the art will understand that other toxins, such as *Bacillus thuringiensis* delta-toxin, and human perforin, can be substituted for aerolysin.

Provided herein are purified proteins (referred to herein as modified aerolysin proteins) that include an aerolysin protein linked to one or more lung cancer-specific agents, and nucleic acid sequences encoding such proteins, which in some examples are used to treat a lung cancer or tumor. The addition of one or more lung cancer-specific agents, such as two, three, or four lung cancer-specific agents, to an aerolysin protein results in a protein that is targeted specifically to lung cancer cells. The lung cancer-specific agents can be attached to any region of aerolysin, or a homolog thereof such as alpha toxin, such as the C-terminus or N-terminus of aerolysin. Such fusion proteins can be generated using standard molecular biology methods. In a particular example, one or more lung cancer-specific agents are attached to an internal amino acid within aerolysin, for example by using a crosslinking agent that reacts with amino groups on the lung cancer-specific agent and with a cysteine located in aerolysin, or by using a homobifunctional-lysine-reactive crosslinking agent using standard methods known in the art.

One example of a lung cancer-specific agent is a lung cancer-specific antibody, such as AFAI that recognizes a non-small lung carcinoma. In one example, AFAI includes at least 80%, at least 85, at least 90%, at least 95%, at least 98, or even at least 99% sequence identity to amino acids 1-135 ofSEQ ID NO: 6, as long as such variants retain the ability to target a toxin, such as alpha toxin or aerolysin, to a lung cancer cell. In a particular example, AFAI includes amino acids 1-135 of SEQ ID NO: 6. Other exemplary lung cancer-specific agents include, but are not limited to, antibodies that recognize carcino embryonic antigen CEA (for example catalog number ab10036 from Abcam Inc., Cambridge, MA), thyroid transcription factor 1 (TTF1) (for example catalog number ab869 from Abcam Inc., Cambridge, MA), and cytokeratin 7 (for example catalog number RDI-PRO61025 from Research Diagnostics Inc., Flanders NJ).

Polypeptides having modified aerolysin activity are disclosed herein. In some examples, modified aerolysin activity is characterized by the ability of a modified aerolysin protein to retain the ability to reduce the volume of a lung tumor, decrease the number of lung cancer or metastasis cells, increase the lysis of lung cancer cells, decrease metastasis from a lung tumor, or combinations thereof. These activities, alone or in combination, can be used to treat a lung tumor or its metastases. In one example, modified aerolysin sequences reduce the volume of a lung tumor by at least 20%, at least 30%, at least 50%, at least 80%, at least 90%, at least 95%, at least 99%, or at least 100% as compared to a volume in the absence of modified aerolysin. In particular examples, modified aerolysin sequences increase the lysis of lung cancer cells *in vivo, in vitro,* or *in situ,* by at least 10%, at least 20%, at least 30%, at least 50%, at least 80%, at least 90%, at least 95%, at least 99%, or at least 100% as compared to an amount of lysis in the absence of modified aerolysin, or in the presence of aerolysin alone (without a lung cancer specific binding agent).

However, the disclosure also encompasses variants, fusions, and fragments of modified aerolysin proteins, such as variants, fusions, and fragments of SEQ ID NO: 6, that retain modified aerolysin activity. A particular example of a variant sequence is one that includes one or more amino acid substitutions, such as at least 2, 3, 4, 5, 6, 10, 12, 15, or even more substitutions.

In a particular example, a variant modified aerolysin protein sequence is one that includes a fragment of a modified aerolysin protein sequence, such as fragments of SEQ ID NO: 6. For example, the disclosure provides modified aerolysin peptides that include at least 15 contiguous amino acids of a disclosed modified aerolysin peptide, such as at least 15 contiguous amino acids of SEQ ID NO: 6. It will be appreciated that the disclosure also provides modified aerolysin peptides that contain an amino acid sequence that is greater than at least 15 amino acid residues of a disclosed modified aerolysin peptide (such as at least 16, 17, 18, 19, 20, 21, 22, 23, 24, 25, 26, 27, 28, 29, 30, 50, 75, 100, 150, 200, 250, 300, 350, 400, 450, or 500 or more amino acid residues).

The disclosure also provides variant modified aerolysin polypeptides that include at least one amino acid insertion, deletion, or substitution, such as 1, 2, 3, 4, 5, or 10 amino acid insertions, deletions, or substitutions, or any combination thereof (such as a single deletion together with 1-10 insertions). In some examples, polypeptides share at least 60%, 65%, 70%, 75%, 80%, 85%, 90%, 95%, 97%, 98%, or 99% sequence identity with a modified aerolysin amino acid sequence (such as SEQ ID NO: 6), as long as the peptide encoded by the amino acid sequence retains modified aerolysin activity. Additional amino acid sequences can be of varying length, such as at least about 5 amino acids, at least abut 10 amino acids, at least about 25 amino acids, at least about 50 amino acids, at least about 100 amino acids, or no more than about 500 amino acids, such as no more than about 250 amino acids, no more than about 100 amino acids, no more than about 75 amino acids, no more than about 50 amino acids, no more than about 25 amino acids, no more than about 15 amino acids, or no more than about 10 amino acids.

One type of variation includes the substitution of one or more amino acid residues, and in some examples no more than 10 amino acids, for amino acid residues having a similar biochemical property, that is, a conservative amino acid substitution. Accordingly, modified aerolysin peptides having at least 1, 2, 3, 4, 5, 10, 20, 30, 40, or 50 conservative substitutions are provided herein. However, more substantial changes can be obtained by selecting substitutions that are less conservative, for example by selecting residues that differ more significantly in their effect on maintaining: (a) the structure of the polypeptide backbone in the area of the substitution, for example, as a sheet or helical conformation; (b) the charge or hydrophobicity of the polypeptide at the target site; or (c) the bulk of the side chain. The substitutions that in general are expected to produce the greatest changes in polypeptide function are those in which: (a) a hydrophilic residue, such as serine or threonine, is substituted for (or by) a hydrophobic residue, such as leucine, isoleucine, phenylalanine, valine or alanine; (b) a cysteine or proline is substituted for (or by) any other residue; (c) a residue having an electropositive side chain, such as lysine, arginine, or histidine, is substituted for (or by) an electronegative residue, such as glutamic acid or aspartic acid; or (d) a residue having a bulky side chain, such as phenylalanine, is substituted for (or by) one not having a side chain, such as glycine. The effects of these amino acid substitutions (or other deletions or additions) can be assessed for peptides having modified aerolysin activity by analyzing the ability of the peptide to target and lyse lung cancer cells, for example as described in Examples 2 and 4-7.

Modified aerolysin proteins variants, fragments, and fusions can be employed in the pharmaceutical compositions, and can include one or more amino acid additions, amino acid deletions, amino acid replacements, or by isostereomer (a modified amino acid that bears close structural and spatial similarity to the original amino acid) substitutions, and isostereomer additions, so long as the resulting modified aerolysin proteins retain modified aerolysin activity. In a particular example, such variants, fragments, and fusions, provide an advantage, such as increasing the solubility of the protein, or easing linking or coupling of the protein. The disclosed modified aerolysin proteins can also be engineered to include additional amino acid segments or polysaccharides. Examples include, but are not limited to, moieties that augment protein stability, manufacture, or delivery within the body to sites appropriate for reducing lung tumors.

Also disclosed are isolated nucleic acid molecules that encode peptides having modified aerolysin activity, for example SEQ ID NO: 5. However, the disclosure also encompasses variants, fusions, and fragments of SEQ ID NO: 5 that retain the ability to encode a protein having modified aerolysin activity. In one example, an isolated nucleic acid molecule encoding a peptide having modified aerolysin activity is operably linked to a promoter sequence, and can be part of a vector. The nucleic acid molecule can be a recombinant nucleic acid molecule that can be used to transform cells. Transformed cells including at least one exogenous nucleic acid molecule encoding a peptide having modified aerolysin activity (such as SEQ ID NO: 5, or fragments, fusions, or variants thereof that retain modified aerolysin activity), are disclosed. Such cells can be used to produce recombinant modified aerolysin proteins, which can be purified and administered to a subject having a lung tumor in a therapeutically effective dose.

The nucleic acid sequences encoding modified aerolysin proteins disclosed herein, such as SEQ ID NO: 5, can contain an entire nucleic acid sequence encoding the protein, as well as a portions thereof that retain the ability to encode a protein having the desired modified aerolysin activity. For example, a modified aerolysin nucleic acid can include at least 15 contiguous nucleotides of a modified aerolysin nucleic acid sequence (such as SEQ ID NO: 5). It will be appreciated that the disclosure also provides isolated nucleic acids that contain a nucleotide sequence that is greater than 15 nucleotides (such as at least 16, 17, 18, 19, 20, 21, 22, 23, 24, 25, 26, 27, 28, 29, 30, 40, 50, 75, 10, 200, 500, 750, 1000, 1250 or more nucleotides) in length of a modified aerolysin sequence shown in SEQ ID NO: 5.

In addition, the disclosure provides isolated modified aerolysin nucleic acid sequences containing a variant modified aerolysin nucleic acid sequence. In particular examples, variants include at least one insertion, deletion, or substitution, such as 1, 2, 3, 4, 5, or 10 insertions, deletions, or substitutions, or any combination thereof (such as a single deletion together with 1-10 insertions) as long as the peptide encoded thereby retains modified aerolysin activity. In some examples, the disclosed isolated nucleic acid molecules share at least 60, 70, 75, 80, 85, 90, 92, 95, 97, 98, or 99% sequence identity with a modified aerolysin sequence (such as a SEQ ID NO: 5), as long as the peptide encoded by the nucleic acid sequence retains modified aerolysin activity. For example, the following variations can be made to the modified aerolysin nucleic acid sequence shown in SEQ ID NO: 5: the "t" at position 48 can be substituted with a "g" "c" or "a"; the "g" at position 102 can be substituted with an "a"; the "c" at position 585 can be substituted with a "t"; the "t" at position 1398 can be substituted with an "c"; and the "t" at position 1533; can be substituted with a "g" "c" or "a". The disclosure also provides isolated nucleic acid sequences that encode for a modified aerolysin peptide, wherein the nucleic acid sequence is at least 12 bases in length (such as at least 13, 14, 15, 16, 17, 18, 19, 20, 25, 30, 40, 50, 60, 100, 250, 500, 750, 1000, 1500, 2000, 3000, 4000, or 5000 bases in length) and hybridizes, under hybridization conditions, to the sense or antisense strand of a nucleic acid encoding the protein. Particular examples of hybridization conditions are provided herein. The effects of these nucleic acid substitutions (or other deletions or additions) can be assessed for sequences that encode for peptides having modified aerolysin activity, for example by determining the ability of the nucleic acid sequence to encode a protein that can specifically lyse lung tumor cells (see Example 2), decreases lung tumor volume or (see Example 5), or both. Modified aerolysin peptides and nucleic acid sequences encoding a modified aerolysin peptide are in some examples produced by standard DNA mutagenesis techniques, for example, M13 primer mutagenesis or PCR. Details of these techniques are provided in Sambrook et al. (ed.), Molecular Cloning: A Laboratory Manual 2nd ed., vol. 1-3, Cold Spring Harbor Laboratory Press, Cold Spring, Harbor, N.Y., 1989, Ch. 15. Nucleic acid molecules can include changes of a coding region to fit the codon usage bias of the particular organism into which the molecule is to be introduced.

The disclosed peptides are in some examples produced using chemical synthesis. Various automatic peptide synthesizers are commercially available and can be used in accordance with known protocols. Chemical synthesis of peptides is described in: S. B. H. Kent, Biomedical Polymers, eds. Goldberg and Nakajima, Academic Press, New York, pp. 213-242, 1980; Mitchell et al., J. Org. Chem., 43:2845-52, 1978; Tam et al., Tet. Letters, 4033-6, 1979; Mojsov et al., J. Org. Chem., 45:555-60, 1980; Tam et al., Tet. Letters, 2851-4, 1981; and Kent et al., Proceedings of the IV International Symposium on Methods of Protein Sequence Analysis, (Brookhaven Press, Brookhaven, N.Y, 1981. In addition, recombinant DNA technology can be employed wherein a nucleotide sequence that encodes one or more modified aerolysin peptides is inserted into an expression vector, transformed or transfected into an appropriate host cell and cultivated under conditions suitable for expression, as disclosed herein.

In some examples, the coding region is altered by taking advantage of the degeneracy of the genetic code to alter the coding sequence in such a way that, while the nucleic acid sequence is substantially altered, it nevertheless encodes a polypeptide having an amino acid sequence identical or substantially similar to the native amino acid sequence. For example, because of the degeneracy of the genetic code, alanine is encoded by the four nucleotide codon triplets: GCT, GCA, GCC, and GCG. Thus, the nucleic acid sequence of the open reading frame can be changed at an alanine position to any of these codons without affecting the amino acid sequence of the encoded polypeptide or the characteristics of the polypeptide. Based upon the degeneracy of the genetic code, nucleic acid variants can be derived from a nucleic acid sequence, for example by using standard DNA mutagenesis techniques or by synthesis of nucleic acid sequences. Thus, this disclosure also encompasses nucleic acid molecules that encode the same polypeptide but vary in nucleic acid sequence by virtue of the degeneracy of the genetic code.

### Treatment of Lung Cancer Using Modified Aerolysin

The modified aerolysin proteins disclosed and discussed above are cytotoxins that specifically target lung cancer cells. Targeting is achieved by including one or more lung cancer-specific binding agents, such as an antibody that recognizes non-small cell lung cancers. The disclosed modified aerolysin proteins can be administered locally or systemically using any method known in the art, to subjects having localized or metastatic lung cancer. In addition, the disclosed modified aerolysin proteins can be administered to a subject for immunostimulatory therapy. However, the present disclosure is not limited to particular means of administration. Due to the specificity of binding and activation of the disclosed modified aerolysin molecules, local and systemic administration should have minimal effect on a patient's normal tissues and ideally produce little to no side effects.

In one example, the disclosed modified aerolysin proteins are injected into the lung tumor (intratumorally) in a subject having lung cancer, such as a localized tumor. Such localized injection and subsequent lysis of lung cancer cells can produce an immunostimulatory effect leading to a decrease or elimination of micrometastatic disease in treated subjects. In this way, systemic disease is treated or reduced through a minimally toxic, locally applied therapy.

In some examples, the disclosed modified aerolysin molecules are administered systemically, for example intravenously, intramuscularly, subcutaneously, or orally, to a subject having lung cancer, such as a metastatic lung tumor. Systemic therapy can also have an immunostimulatory anti-tumor effect.

An additional method for systemically treating lung cancer in a subject is also disclosed. In some examples, the method includes removing lung cancer cells from the subject having lung cancer, such as a metastatic lung tumor. Established lung cancer cell lines can also be used, such as non small cell lung cancer cell lines, for example A549, NCI-H23, NCI-H157, NCI-H520 and NCI-H522 and small cell lung cancer cell lines such as NCI-1469, SCC-9, NCI-H146 and NCI-H345. The removed cells or cell lines are incubated or contacted with a modified aerolysin protein. This incubation results in lysis of the cells by the modified aerolysin protein, and production of a cell lysate that is administered to the subject. In one example, the method further includes administration of immunostimulatory factors, lysates from lung cancer cells engineered to produce immunostimulatory factors, or irradiated lung cancer cells (including lung cancer cells engineered to produce immunostimulatory factors). Examples of immunostimulatory factors include, but are not limited to: granulocyte macrophage colony stimulatory factor (GM-CSF); members of the interleukin family of proteins such as but not limited to interleukin-2 and interleukin-6, granulocyte colony stimulatory factor (G-CSF); and members of interferon family such as interferon alpha, beta or gamma. Administration of such materials to a subject can be simultaneous with the cell lysate (co-administration), before administration of the cell lysate, subsequent to administration of the cell lysate, or combinations thereof.

The therapeutic agents, such as modified aerolysin proteins and nucleic acids, can be part of an *in vitro* solution, an *in vivo* expression system, or *in situ* with a host tissue or subject. In some examples, in forming a pharmaceutical composition for reducing lung tumors in a subject, one or more modified aerolysin proteins (or nucleic acid molecules that encode such proteins), alone or in combination with other agents, is utilized. For example, the therapeutic agent can be administered in a pharmaceutically acceptable carrier. Furthermore, the therapeutic agent can be administered with additional therapeutic agents (such as before, during of after administration of a modified aerolysin protein or nucleic acid molecule), such as those that reduce the production of antibodies to the administered modified aerolysin proteins (for example steroids) and other anti-tumor agents. The disclosed modified aerolysin proteins can be administered as a single modality therapy or used in combination with other therapies, such as radiation therapy, chemotherapy, or surgery.

In some examples, the method includes contacting a cell, such as a lung cancer cell, with a therapeutically effective amount of a modified aerolysin protein. In particular examples, the cell is incubated with the protein for a time sufficient to allow the modified aerolysin protein to bind and lyse the cell.

In some examples, the method includes expressing a modified aerolysin nucleic acid sequence in a cell, such as a bacterial cell, yeast cell, or mammalian cell, in order to produce recombinant modified aerolysin protein. The recombinant modified aerolysin protein can be purified from the cell or extracellular medium using methods known in the art. The purified protein can then be contacted with a cell, for example by administering the protein to a subject using the methods described above, for example to treat a lung tumor.

In particular examples, the cell is present in a subject, such as a subject having a lung cancer, and the method includes administering a therapeutically effective amount of the modified aerolysin protein to the subject. In one example, the lung-cancer status of the subject, such as a human subject, is determined prior to administering a therapeutically effective amount of modified aerolysin. This allows one, such as a physician, to determine whether the subject has a lung cancer that could benefit from the disclosed therapies. For example, if the subject is determined to have a non-small cell lung cancer, the subject can be administered a modified aerolysin, such as AFAI-aerolysin, in order to reduce the presence of the tumor in the subject.

In one example, such administration decreases the volume of a lung tumor or a metastatic tumor, or both. Decreasing the volume of a lung tumor or a metastatic tumor does not require a 100% reduction in the volume, and in some examples includes decreasing the volume by at least 10%, for example by at least 20% or more as compared to a volume in the absence of the therapeutic agent. In one example, such administration enhances the lysis of lung cancer cells, or metastatic tumor cells, or both. Increasing the lysis of lung cancer cells, or metastatic tumor cells, does not require a 100% lysis, and in some examples includes increasing the lysis by at least 10%, for example by at least 20% or more as compared to an amount of lysis in the absence of the therapeutic agent. In addition, the disclosed methods can result in a decrease in the symptoms associated with a lung tumor or a metastatic lung tumor.

Disclosure of certain specific examples is not meant to exclude other embodiments. In addition, any treatments described herein are not necessarily exclusive of other treatment, but can be combined with other bioactive agents or treatment modalities.

### EXAMPLE 1

### Fusion of single domain antibody AFAI to the large lobe of aerolysin

This example describes methods used to produce protein (SEQ ID NO: 6) that includes AFAI (an antibody specific for non-small cell lung cancer cells) fused to the N-terminus of the large lobe of aerolysin. However, one skilled in the art will appreciate that similar methods can be used to attach AFAI to the C-terminus of the large lobe of aerolysin. For example, amino acids 1-135 of SEQ ID NO: 6 (AFAI) can be linked to the C-terminus of amino acids 136-526 of SEQ ID NO: 6 (aerolysin).

Antibodies produced by *Tylopoda* (camels, dromedaries and llamas) produce a unique antibody class formed by heavy-chains only (for example see Muyldermans et al. Trends Biochem. Sci. 26:230-5, 2001). The variable domain of this type of antibody is referred to as VHH. The AFAI antibody is a homodimeric heavy-chain antibody that was obtained as follows. The VHH repertoire of a non-immunized llama was amplified by PCR and cloned. The AFAI VHH or single domain antibody recognized an antigen that is transiently expressed during mitosis, and is up-regulated by EGF. The antibody was selected by phage display of the llama library and panning with the lung cancer cell line A549 using human fibroblasts as the reference or subtracting cells.

The DNA encoding AFAI, a VHH which binds to an unidentified epitope on non-small cell lung cancer cells, was fused to the DNA encoding the large lobe of aerolysin (amino acids 136-526 of SEQ ID NO: 6), which can form channels in cell membranes. The fused DNA was then cloned into a pUC-based vector called pSJF2, to add an *E. coli* OmpA signal sequence.

AFAI was PCR-amplified with the following primers: GGT GCG CAG GCC GTC TTC GAG GTC CAG CTG CAG GCG (SEQ ID NO: 1) and CGT ACG GTG CAC TGA GGA GAC GGT GAC CTG (SEQ ID NO: 2) to introduce 5'-Bbs1 at 5' and 3'-Apal1 at 3' (AFAI and EC1 have the same 3'-sequence). The large lobe of aerolysin was PCR-amplified with the following primers: CGT ACG GTG CAC CCT GTT ACC GGT GAA ATA (SEQ ID NO: 3) and TTC TGT AAG CTT TGA TTG GCA GCA GGG GA (SEQ ID NO: 4) to introduce a 5'-Apal1 site and 3 stop codons preceding a 3'-HindIII at 3' site. The PCR products were purified with the QIAquick kit (Qiagen) and digested with both with Apal1. The digested products were purified with the QIAquick kit, and ligated in a mixture containing 2.0 µl 20ng/ul digested AFAI, 3.5 µl 42 ng/µl digested aerolysin, 2.0 µl 5 x ligase buffer (Invitrogen), 2.0 µl water and 0.5 µl ligase (Invitrogen, 1 unit/µl) at room temperature for 2 hours.

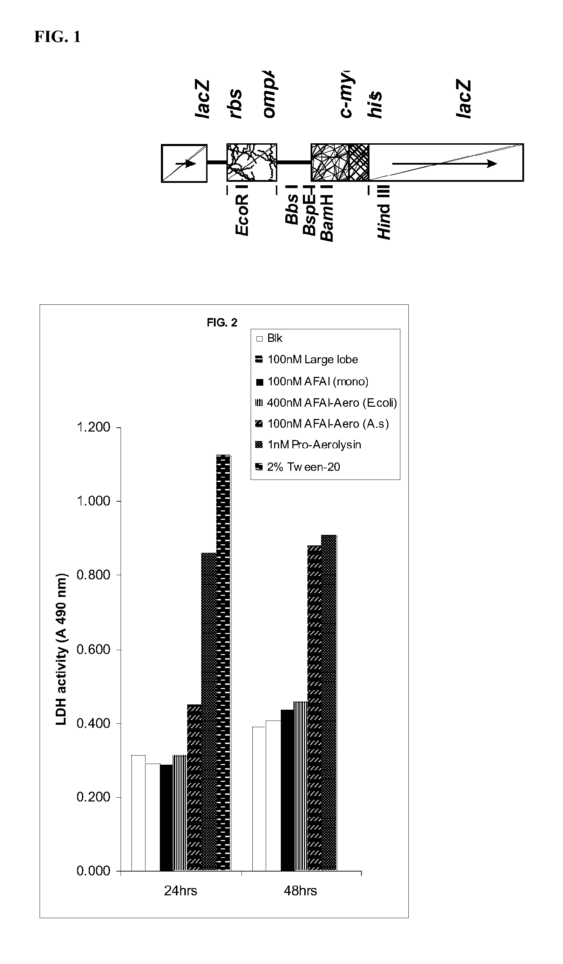

The ligated products were PCR-amplified with SEQ ID NOS: 2 and 4 in 50 µl reaction buffer containing 1 µl of ligated DNA, 2 µM of each primer, 2.5 mM Mg and 1 µl ligase (Roche) under the following PCR conditions: 5 minutes at 95°C; 7 cycles of 95°C for 1 minute, 63°C for 30 seconds, 58°C for 50 seconds, 72°C for 1 minute; 23 cycles of 95°C for 1 minute, 63°C for 30 seconds, 72°C for 1 minute; 72°C for 10 minutes. The resulting PCR product was digested with Bbs1 and HindIII, and the digested products purified with the QIAquick kit and ligated into pSJF2 (FIG. 1) which was gel purified following digestion with Bbs1 and HindIII. The ligation mixture containing 1 µl digested pSJF2 (10 ng/µl), 0.5 µl digested sdAb-aerolysin fusion (20 ug/µl), 2 µl 5 x ligase buffer, 6 µl water, 0.5 µl T4 DNA ligase (Invitrogen, 1 unit/µl) was incubated at room temperature for 1 hour.

The resulting ligation product (0.5 µl) was transformed into competent *Escherichia coli* TG1, and the cells plated on LB containing carbenicillin and screened for clones that carry the correct plasmid. AFA1-aerolysin was digested from the pSJF2 vector using EcoRI and Hind III enzymes, and ligated into the expression vector pMMB67EH (Furste et al., Gene 48:119-31, 1986). This ligated product was transformed into competent *E. coli* DH5alpha and clones screened for the correct plasmid by PCR. The pMMB67EH:AFA1-aerolysin was transconjugated into *A. salmonicida* CB3 cells with the aid of *E. coli* helper strain MM297, and the clones screened by PCR.

CB3 cells containing the plasmid were grown overnight as previously described (Buckley, Cell. Biol. 68:221-4, 1990), subbed and grown to an OD₆₀₀ of approximately 1.0. The cells were induced by adding 1 mM IPTG. Four hours after induction, the cells were removed by centrifugation and the culture supernatant concentrated approximately 50-fold by filtration, using a 10,000 Mwt cutoff filter. The concentrate was centrifuged to remove insoluble material and then applied to a Sephadex G25 column to exchange the buffer to 300 mM NaCl, 20 mM phosphate, pH 6.0. The peak containing the protein fusion was applied to a hydroxyapatite column and the protein eluted with a phosphate gradient. The fusion protein (SEQ ID NO: 6, encoded by SEQ ID NO: 5) was recovered from the eluting peak by adding ammonium sulfate to 60 % of saturation. Following centrifugation, the protein was dissolved in 20 mM HEPES, pH 7.4 and applied to a DEAE Sepharose column. The fusion product eluted as a single peak upon application of a gradient of NaCl.

### EXAMPLE 2

### Cytotoxicity of AFAI-aerolysin

This example describes methods used to determine the cytotoxicity of the AFAI-aerolysin hybrid described in Example 1, towards A549 (non-small cell lung carcinoma) cells. Such methods can be used to test the cytotoxicity of any aerolysin molecule fused to a lung-cancer cell specific agent, such as an antibody.

The cytotoxicity of the AFAI-aerolysin hybrid towards A549 cells was determined by measuring the amount of release of a cytoplasmic enzyme (lactate dehydrogenase, LDH) in the presence of the AFAI-aerolysin hybrid in comparison to control molecules. Cells were induced with EGF at 10ng/ml for 48 hours in 1 ml of DMEM + 5% FBS in 24 well plates (10⁴ cells/well). Just prior to the experiment, to reduce exogenous serum LDH, cells were washed once with medium + 1% inactivated FBS and incubated in 500 µl of DMEM + 1% inactivated FBS with hybrids (100 nM and 400 nM AFAI-aerolysin from *A. salmonicida* and *E. coli,* respectively) and controls (1 nM proaerolysin, 100 nM large lobe of aerolysin). Quadruplicate samples of culture medium were collected at 5 hours, 24 hours, 30 hours and 48 hours and tested for LDH activity using the LDH cytotoxicity detection kit from Roche (catalog number 1 644 793). As shown in FIG. 2, only the AFAI-aerolysin hybrids (as well as the detergent Tween-20) increased LDH activity. This demonstrates that the AFAI-aerolysin hybrids can lyse lung cancer cells with specificity.

The cytotoxicity of the AFAI-aerolysin hybrid towards A549 cells was also determined by examining the cells by light microscopy. A549 cells were grown and treated as described above, and subsequently analyzed by microscopy. It was observed that proaerolysin and AFAI-aerolysin lysed A549 cells, but EC-aerolysin and the large lobe of aerolysin do not.

### EXAMPLE 3

### Immunocytostaining of A549 cells with AFAI-Aerolysin

This example describes methods used to further demonstrate the targeting of the AFAI-aerolysin molecule specifically to lung cancer cells. Similar methods can be used to screen other modified aerolysin molecules.

A549 cells were seeded in presence of EGF (10 ng/ml) on cover slips in 6 well plates at 10⁵ cells per well for 48 hours. Cells were fixed with 4% formaldehyde for 10 minutes, washed with PBS + 0.05% Tween-20 and permeabilized with NP 40 for 30 minutes. Non-specific sites were blocked with 3% BSA for 1 hour the first antibody, AFAI-aerolysin, was added at 5 µg/ml for 4 hours. Cells were subsequently incubated with a second, rabbit polyclonal anti-aerolysin used at 1/1500, and a third antibody, anti-rabbit IgG-Alexa Fluor 546 used at 1/200, for 1 hour and 30 minutes, respectively. Between each step, cells were washed 3 times with PBS + 0.05% Tween-20 to remove unbound antibodies. Nuclei and cell membranes were stained with DAPI (1/10⁵) and DiOC₅ (1/10⁵), respectively, for 15 minutes. Staining with AFAI-aerolysin, the nuclear staining and the membrane staining were observed using a fluorescence microscope equipped with appropriate filters.

AFAI-aerolysin was observed to bind strongly to A549 cells. The intensity of the staining relative to that observed for proaerolysin indicated that the AFAI antigen may be more abundant than the GPI-anchor recognized by proaerolysin. No staining was observed when only the detecting antibodies were employed.

### EXAMPLE 4

### in vitro and in vivo Toxicity of Modified Aerolysin

This example describes methods used to determine the *in vitro* and *in vivo* toxicity of a modified aerolysin molecule.

To determine *in vitro* toxicity, a cell viability assay can be performed as follows. E14 mouse T-cell lymphoma cells (ATCC TIB-39) are cultured at 10⁵ cells per well in MTS/PMS Cell Titer 96 (Promega). Modified aerolysin molecules at 1 x 10⁻¹³ M- 1 x 10⁻⁷ M are added, and incubated with the cells for 4 hours at 37°C. Cell viability is subsequently determined by reading the plate on a plate reader, as directed by the manufacturer of the MTS/PMS kit.

To determine *in vivo* toxicity, modified aerolysin molecules are administered to mice intravenously. Wild-type proaerolysin is highly toxic to mice; a dose of 1 µg causes death within one hour and the LD₁₀₀ at 24 hours (the dose that kills 100% of animals within 24 hours) following a single IV injection was 0.1 µg.

### EXAMPLE 5

### Reduction of Tumor Cell Volume

The following methods can be used to demonstrate that the modified aerolysin molecules decrease the volume of non-small cell lung carcinomas. Mice having a human lung cancer xenograft(s) can be generated using the method of Forsberg et al. (British J. Cancer 85:129-36, 2001), with the following modifications. Briefly, the xenograph is established mice (such as SCID or Balb/c nu/nu mice). However, it is not necessary to inject peripheral blood mononuclear cells if modified aerolysin is used. The xenograph can be allowed to grow for a period of time prior to administration of modified aerolysin (such as 5 days). Alternatively, modified aerolysin can be administered at the same time as the cancer cells, and growth of the xenograph monitored in the absence of further administration of modified aerolysin (to determine if xenograph growth can be prevented). The development of the lung cancer xenograph can be compared to administration of modified aerolysin in SN12C bearing mice (control mice which have a human renal carcinoma xenograft).

The amount of modified aerolysin administered can be 0.1-10 times the LD₁₀₀ dose determined in EXAMPLE 4. Following injection (such as 48 hours later), tumors are harvested, fixed and stained with H&E, and for Ki-67 (proliferative index) and Tunel (apoptotic index). The percent of tumor within sample is determined by calculating the ratio of viable tumor to total tumor area following image analysis of thin tumor sections.

Administration of modified aerolysin should reduce lung tumor cell volume to a greater extent than the control tumors (SN12C mice).

### EXAMPLE 6

### Determination of Modified Aerolysin Antigenicity

This example provides methods to determine if modified aerolysin proteins disclosed herein are antigenic. In addition, methods to reduce potential antigenicity are disclosed.

As described in the Examples above, modified aerolysin can be administered to a subject by intratumoral injection, intravenous (iv), intramuscular, oral, etc., as a systemic therapy for lung cancer. However, systemic administration of the modified aerolysin peptides disclosed herein may result in the development of a neutralizing antibody response that would limit repeat dosing.

The kinetics and magnitude of the antibody response to any of the aerolysin variants disclosed herein can be determined as follows. For example, the antigenic response to modified aerolysin can be determined in immunocompetent mice, to develop a dosing regimen that can be used in an immunocompetent human. Immunocompetent mice (C57-BL6) are administered iv doses of modified aerolysin both daily x 5 and weekly x 3 at a dose range from 0.1 µg to 5 µg. Mice are sacrificed at varying intervals (such as following single dose, following multiple doses) and serum obtained. An ELISA-based assay can be used to detect presence of anti-aerolysin antibodies. In this assay, a defined quantity of aerolysin is fixed to the polystyrene surface in 96-well plates. Following adequate blocking with bovine serum albumin (BSA), serum from mice exposed to aerolysin is added to the wells at varying dilutions. After a defined incubation time, wells are washed, and alkaline phosphatase linked goat-anti-mouse secondary antibody is added, followed by substrate. The amount of antibody present is determined by measuring absorbance in a spectrophotometer, which permits determination of the time course and magnitude of the antibody response by varying schedules and doses of iv modified aerolysin.

One method to overcome the potential antigenicity of the disclosed modified aerolysin toxins is to sequentially administer structurally related protoxins that are similarly targeted to lung tumors by an antibody, but which are not recognized by proaerolysin antibodies. Examples of such protoxins include, but are not limited to, *Clostridium septicum* alpha toxin (Ballard et al., Infect. Immun. 63:340-4, 1995; Gordon et al. J. Biol. Chem. 274:27274-80, 1999; Genbank Accession No. S75954), *Bacillus Thuringiensis* delta-toxin (Genbank Accession No. D00117), and human perforin (Genbank Accession No. NM005041). While mechanistically similar to aerolysin, these protoxins have different peptide sequences such that antibodies specific to proaerolysin would not recognize them. These protoxins have been cloned and recombinant forms produced (Imagawa et al., FEMS. Microbiol. Lett. 17:287-92, 1994; Meza et al. FEMS Microbiol. Lett. 145:333-9, 1996).

Another method for reducing the systemic immune response is to administer immunosuppressive therapies. Examples of immunosuppressive therapies include, but are not limited to, systemic or topical corticosteroids (Suga et al., Ann. Thorac. Surg. 73:1092-7, 2002), cyclosporin A (Fang et al., Hum. Gene Ther. 6:1039-44, 1995), cyclophosphamide (Smith et al., Gene Ther. 3:496-502, 1996), deoxyspergualin (Kaplan et al., Hum. Gene Ther. 8:1095-1104, 1997) and antibodies to T and/or B cells [such as anti-CD40 ligand, anti CD4 antibodies, anti-CD20 antibody (Rituximab)] (Manning et al., Hum. Gene Ther. 9:477-85, 1998). Such agents can be administered before, during, or subsequent to administration of modified aerolysin molecules or cell lysates produced by incubation with aerolysin.

### EXAMPLE 7

### Induction of a Systemic Immunostimulatory Response

This example provides methods that can be used to demonstrate that aerolysin-mediated cell lysis produces a systemic immunostimulatory effect. Such a systematic immunostimulatory effect resulting from a direct administration of the toxins disclosed herein to the lung tumor, would provide both local therapy for lung cancer, while simultaneously induce a systemic antitumor effect against occult micrometastatic disease. Alternatively or in addition, subjects can be vaccinated with modified aerolysin-lysed lung cancer cells in the presence or absence of cytokines, such as GMCSF, to treat recurrent or initial metastatic disease.

### Administration of Lung Tumor Cells Lysed with Modified αerolysin

To demonstrate that hybrid-aerolysin treated cells stimulate a systemic immune response in a subject, the following methods can be used. Briefly, subjects (such as immunocompetent mice or a human subject having lung cancer) are administered lung tumor cells (such as A549 human lung tumor cells or other human lung cancer cell lines approved for administration to patients, or lung cancer cells obtained from the subject having lung cancer) which have been lysed with one or more modified aerolysin molecules disclosed herein. To determine whether a systemic immune response occurs, mice that have been administered lysed lung cancer cells can be rechallenged with the same cells and growth of these inoculated tumor cells measured. For example, mice having a lung tumor xenograph are subcutaneously injected with aerolysin-lysed A549 cells. To accomplish this, 10⁷ A549 cells are lysed by incubation with aerolysin for 1 hour at 37°C in sterile phosphate buffered saline (PBS). Animals are administered two weekly injections of this cell lysate to stimulate an immune response to the A549 cells. Animals are subsequently rechallenged with a subcutaneous injection of A549 cancer cells one week after the second lysate inoculation. Control subjects receive a similar number of cells that have been lysed by freezing and thawing, or that have been treated with radiation to induce apoptosis. Another control group will receive only the modified aerolysin peptide. Tumor growth is compared between the groups using the methods described in the above Examples.

For human subjects, lung cancer cells can be obtained from patient at time of biopsy or human lung cancer cell lines can be used. Examples of human lung cancer cell lines include, but are not limited to: non small cell lung cancer cell lines such as A549, NCI-H23, NCI-H157, NCI-H520 and NCI-H522 and small cell lung cancer cell lines such as NCI-1469, SCC-9, NCI-H146 and NCI-H345. Approximately 10⁷-10⁸ cells from either source (patient or cell lines) are incubated with 1 µg of aerolysin (wild-type or modified aerolsyin) for 1 hour at 37°C in sterile PBS. The resulting lysate is mixed thoroughly using vigorous pipetting, and suspension is administered to a subject via subcutaneous injection of ~ 0.5 ml. Patients can be treated weekly for 3 doses. Patients are closely monitored with weekly physical exams. Immune response to subcutaneous aerolysin can be monitored by assaying presence of antibodies to aerolysin, and assaying for B and T cell response using known methodology.

Alternatively, or in addition, lung cancer cells from a patient or lung cancer cell lines engineered to express immunostimulatory proteins (such as GM-CSF) are incubated with aerolysin (wild-type or modified aerolysin) and used to produce the lysate. Lysed lung cancer cells can be co-administered with irradiated lung cancer cells producing immunostimulatory molecules. Irradiation induces cells to undergo apoptosis. The combination of cells killed by cytolysis and cells killed by induction of apoptosis is expected to produce superior immunostimulatory effects. In one example, a subject is co-administered aerolysin-lysed lung cancer cells mixed with immunostimulatory protein. In another example, aerolysin-lysed lung cancer cells are administered subcutaneously, and an immunostimulatory protein, such as GM-CSF, interleukins, interferons, G-CSF (Dranoff et al. Proc. Natl. Acad. Sci. USA 90:3539-43, 1993) is systemically administered.

### Intratumoral Administration of Modified Aerolysin

Another method that can be used to stimulate a systemic immune response in a subject having lung cancer is to administer modified aerolysin toxins disclosed herein directly into the lung (or the tumor itself) of a human subject having a lung tumor. It is expected that cytolysis following intratumoral injection of modified aerolysin toxins will provoke an immune response to lung tumor antigens.

To determine the appropriate dose to be administered intratumorally, a dose finding clinical trial is performed. Patients can receive multiple injections (20-80) at predefined sites to encompass the entire lung or tumor(s). The dose per injection will be determined by dividing total dose by total number of injections. Patients will be examined weekly for signs of toxicity. MRI of the lung can be used to monitor direct treatment effect on tumor size.

### EXAMPLE 8

### Crosslinking of Lung-Specific Binding Agents to a Toxin

As described in Example 1, a lung cancer specific binding agent, such as an antibody, can be conjugated to an N- or C-terminus of a toxin, such as aerolysin or alpha toxin. This example describes methods that can be used to crosslink lung cancer specific binding agents to any region of the toxin, such as a Cys residue in the toxin.

Lung cancer specific binding agents, such as AFAI (amino acid 1-135 of SEQ ID NO: 6) can be attached to various portions of a toxin protein such as aerolysin. Ideally however, such placement will not significantly interfere with the ability of the toxin to insert into the membrane to form a pore.

Lung cancer specific binding agents, such as antibodies or FAB fragments, can be attached to a toxin by covalent crosslinking (for example see Woo et al., Arch. Pharm. Res. 22(5):459-63, 1999 and Debinski and Pastan, Clin. Cancer Res. 1(9):1015-22, 1995). Crosslinking can be non-specific, for example by using a homobifunctional-lysine-reactive crosslinking agent, or it can be specific, for example by using a crosslinking agent that reacts with amino groups on the antibody and with cysteine located in aerolysin (such as amino acids Cys215 or Cys220 of SEQ ID NO: 6).

As an alternative to recombinantly producing a modified aerolysin, the lung-specific binding agents can be coupled directly to a C-terminal carboxyl of an aerolysin by the addition of a Cys to the C-terminus of the aerolysin, then crosslinking this Cys to the lung cancer specific binding agent. This coupling will produce a modified aerolysin protein in which the lung cancer specific binding agent is attached to the C-terminus of aerolysin. Similar methods can be used to produce a lung cancer specific binding agent attached to the N-terminus of aerolysin.

In other examples, a cysteine residue is introduced into a lung cancer specific binding agent and the agent attached to aerolysin via a disulfide bridge.

The resulting modified aerolysin proteins that include a lung cancer specific binding agent are tested *in vitro* and *in vivo* for their ability to lyse lung cancer cells, for example using the methods described in Examples 2 and 4-7. Using these methods, regions of aerolysin to which a lung cancer specific binding agent can be attached without interfering with channel formation by the toxin are identified.

### EXAMPLE 9

### Alpha Toxin fused to a Lung Cancer Specific Binding Agent

This example describes methods that can be used to generate a modified aerolysin molecule that includes alpha toxin fused to a lung cancer specific binding agent, such as AFAI (amino acids 1-135 of SEQ ID NO: 6).

Such modified alpha toxin molecules can be produced recombinantly, for example using methods similar to those described in Example 1. For example, AFAI (amino acids 1-135 of SEQ ID NO: 6) can be linked to an N- or C-terminus of alpha toxin (such as SEQ ID NO: 8). In addition, AFAI (amino acids 1-135 of SEQ ID NO: 6) can be conjugated to an alpha toxin using the methods described in Example 8. For example, one or more lung cancer specific binding agents, such as AFAI, can be cross-linked to Cys12, Cys20, or Cys86 of SEQ ID NO: 8.

### EXAMPLE 10

### Recombinant Expression of Proteins

With publicly available aerolysin and alpha toxin cDNA and corresponding amino acid sequences, as well as the disclosure herein of aerolysin and alpha toxin variants, fragments and fusions, the expression and purification of any modified aerolysin protein by standard laboratory techniques is enabled. The purified protein can be used for patient therapy. One skilled in the art will understand that the disclosed modified aerolysin toxins can be produced in any cell or organism of interest, and purified prior to administration to a subject.

Methods for producing recombinant proteins are well known in the art. Therefore, the scope of this disclosure includes recombinant expression of any protein. For example, see U.S. Patent No: 5,342,764 to Johnson et al.; U.S. Patent No: 5,846,819 to Pausch et al.; U.S. Patent No: 5,876,969 to Fleer et al. and Sambrook et al. (Molecular Cloning: A Laboratory Manual, Cold Spring Harbor, New York, 1989, Ch. 17, herein incorporated by reference).

The nucleic acid sequence encoding a modified aerolysin toxin can be under the control of a suitable promoter. Suitable promoters include, but are not limited to, the gene's native promoter, retroviral LTR promoter, or adenoviral promoters, such as the adenoviral major late promoter; the CMV promoter; the RSV promoter; inducible promoters, such as the MMTV promoter; the metallothionein promoter; heat shock promoters; the albumin promoter; the histone promoter; the α-actin promoter; TK promoters; B 19 parvovirus promoters; and the ApoAI promoter. In one example, the promoter is a lung-specific promoter. However, the disclosure is not limited to specific promoters.

### EXAMPLE 11

### Peptide Modifications

Modified aerolysin proteins which are targeted to a lung cancer cell, can be modified using a variety of chemical techniques to produce derivatives having essentially the same activity as the unmodified peptides, and optionally having other desirable properties (such as reduced antigenicity). For example, carboxylic acid groups of the peptide, whether carboxyl-terminal or side chain, can be provided in the form of a salt of a pharmaceutically-acceptable cation or esterified to form a C₁-C₁₆ ester, or converted to an amide of formula NR₁R₂ wherein R₁ and R₂ are each independently H or C₁-C₁₆ alkyl, or combined to form a heterocyclic ring, such as a 5- or 6- membered ring. Amino groups of the peptide, whether amino-terminal or side chain, can be in the form of a pharmaceutically-acceptable acid addition salt, such as the HCl, HBr, acetic, benzoic, toluene sulfonic, maleic, tartaric and other organic salts, or may be modified to C₁-C₁₆ alkyl or dialkyl amino or further converted to an amide.

Hydroxyl groups of the peptide side chain can be converted to C₁-C₁₆ alkoxy or to a C₁-C₁₆ ester using well-recognized techniques. Phenyl and phenolic rings of the peptide side chain can be substituted with one or more halogen atoms, such as F, Cl, Br or I, or with C₁-C₁₆ alkyl, C₁-C₁₆ alkoxy, carboxylic acids and esters thereof, or amides of such carboxylic acids. Methylene groups of the peptide side chains can be extended to homologous C₂-C₄ alkylenes. Thiols can be protected with any one of a number of well-recognized protecting groups, such as acetamide groups. Those skilled in the art will also recognize methods for introducing cyclic structures into the peptides disclosed herein to select and provide conformational constraints to the structure that result in enhanced stability. For example, a carboxyl-terminal or amino-terminal cysteine residue can be added to the peptide, so that when oxidized the peptide will contain a disulfide bond, generating a cyclic peptide. Other peptide cyclizing methods include the formation of thioethers and carboxyl- and amino-terminal amides and esters.

To maintain a functional peptide, particular peptide variants will differ by only a small number of amino acids. Such variants can have deletions (for example of 1-3 or more amino acids), insertions (for example of 1-3 or more residues), or substitutions that do not interfere with the desired activity of the peptide. Substitutional variants are those in which at least one residue in the amino acid sequence has been removed and a different residue inserted in its place. In particular examples, such variants have amino acid substitutions of single residues, for example 1, 3, 5 or even 10 substitutions in a protein.

Peptidomimetic and organomimetic embodiments are also disclosed herein, whereby the three-dimensional arrangement of the chemical constituents of such peptido- and organomimetics mimic the three-dimensional arrangement of the peptide backbone and component amino acid sidechains in the peptide, resulting in such peptido- and organomimetics of a modified aerolysin toxin that has the ability to lyse lung cancer cells. For computer modeling applications, a pharmacophore is an idealized, three-dimensional definition of the structural requirements for biological activity. Peptido- and organomimetics can be designed to fit each pharmacophore with current computer modeling software (using computer assisted drug design or CADD). See Walters, "Computer-Assisted Modeling of Drugs", in Klegerman & Groves, eds., 1993, Pharmaceutical Biotechnology, Interpharm Press: Buffalo Grove, IL, pp. 165-174 and Principles of Pharmacology (ed. Munson, 1995), chapter 102 for a description of techniques used in CADD.

### EXAMPLE 12

### Generation and Expression of Fusion Proteins

This example describes methods that can be used to add amino acids to the disclosed modified aerolysin molecules.

Methods for making fusion proteins are well known to those skilled in the art. For example U.S. Patent No. 6,057,133 to Bauer et al. (herein incorporated by reference) discloses methods for making fusion molecules composed of human interleukin-3 (hIL-3) variant or mutant proteins functionally joined to a second colony stimulating factor, cytokine, lymphokine, interleukin, hematopoietic growth factor or IL-3 variant. U.S. Patent No. 6,072,041 to Davis et al. (herein incorporated by reference) discloses the generation of fusion proteins comprising a single chain Fv molecule directed against a transcytotic receptor covalently linked to a therapeutic protein.

Similar methods can be used to generate fusion proteins including aerolysin (or variants, fragments, etc. thereof) linked to other amino acid sequences, such as a lung cancer specific binding agent (for example AFAI). Linker regions can be used to space the two portions of the protein from each other and to provide flexibility between them. The linker region is generally a polypeptide of between 1 and 500 amino acids in length, for example less than 30 amino acids in length, for example between 5 and 20 amino acids in length. The linker joining the two molecules can be designed to (1) allow the two molecules to fold and act independently of each other, (2) not have a propensity for developing an ordered secondary structure which could interfere with the functional domains of the two proteins, (3) have minimal hydrophobic or charged characteristic which could interact with the functional protein domains and (4) provide steric separation of the two regions. Typically surface amino acids in flexible protein regions include Gly, Asn and Ser. Other neutral amino acids, such as Thr and Ala, can also be used in the linker sequence. Additional amino acids can be included in the linker due to the addition of unique restriction sites in the linker sequence to facilitate construction of the fusions. Other moieties can also be included, as desired. These can include a binding region, such as avidin or an epitope, such as a polyhistadine tag, which can be useful for purification and processing of the fusion protein. In addition, detectable markers can be attached to the fusion protein, so that the traffic of the fusion protein through a body or cell can be monitored conveniently. Such markers include radionuclides, enzymes, fluorophores, and the like.

Fusing of an aerolysin nucleic acid sequence (or variant or fragment thereof) with a nucleic acid sequence encoding another protein can be accomplished by the use of intermediate vectors. Alternatively, one gene can be cloned directly into a vector containing the other gene. Linkers and adapters can be used for joining the nucleic acid sequences, as well as replacing lost sequences, where a restriction site was internal to the region of interest. Genetic material (DNA) encoding one polypeptide, peptide linker, and the other polypeptide is inserted into a suitable expression vector which is used to transform prokaryotic or eukaryotic cells, for example bacteria, yeast, insect cells or mammalian cells. The transformed organism is grown and the protein isolated by standard techniques, for example by using a detectable marker such as nickel-chelate affinity chromatography, if a polyhistadine tag is used. The resulting product is therefore a new protein, a fusion protein, which includes modified aerolysin joined by a linker region to a second protein. To confirm that the fusion protein is expressed, the purified protein is subjected to electrophoresis in SDS-polyacrylamide gels, and transferred onto nitrocellulose membrane filters using established methods. The protein products can be identified by Western blot analysis using antibodies directed against the individual components, such as a polyhistadine tag and PA.

### EXAMPLE 13

### Pharmaceutical Compositions and Modes of Administration

The pharmaceutically effective carriers useful herein are conventional. Remington's Pharmaceutical Sciences, by Martin, Mack Publishing Co., Easton, PA, 15th Edition (1975), describes compositions and formulations suitable for pharmaceutical delivery.

In an example in which a modified aerolysin protein, such as SEQ ID NO: 6, is administered to a subject, the protein is delivered by any route used by those in the art. Examples include, but are not limited to: intravenously, intratumorally, intradermal, intraperitoneal, intramuscularly, subcutaneous injection, transdermal, and orally. Proteins can be administered by any convenient route, for example by infusion or bolus injection, by absorption through epithelial or mucocutaneous linings (such as oral mucosa, rectal, vaginal and intestinal mucosa, etc.) and may be administered together with other biologically active agents. Administration can be systemic or local.

The present disclosure also provides pharmaceutical compositions that include a therapeutically effective amount of a modified aerolysin protein alone or with a pharmaceutically acceptable carrier. Furthermore, the pharmaceutical compositions or methods of treatment can be administered in combination (or separately) with one or more other therapeutic treatments. Examples of other therapeutics include, but are not limited to anti-tumor agents, cell lysates (such as those generated by incubation with a modified aerolysin protein), non-lysed cells (such as those that have been killed by radiation), immunosuppressants (such as steroids), or cytokines (such as GM-CSF). Embodiments of the disclosure including medicaments can be prepared with conventional pharmaceutically acceptable carriers, adjuvants and counterions as would be known to those of skill in the art.

A modified aerolysin protein can be administered in combination with at least one, for example one or more pharmaceutically effective carriers, such as a pharmaceutically and physiologically acceptable fluid. Examples of pharmaceutically effective carriers include, but are not limited to water, physiological saline, balanced salt solutions, aqueous dextrose, sesame oil, glycerol, ethanol, combinations thereof, or the like, as a vehicle. The carrier and composition can be sterile, and the formulation suits the mode of administration. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The composition can be a liquid solution, suspension, emulsion, tablet, pill, capsule, sustained release formulation, or powder. For solid compositions (such as a powder, pill, tablet, or capsule forms), conventional non-toxic solid carriers can include, for example, pharmaceutical grades of mannitol, lactose, starch, sodium saccharine, cellulose, magnesium carbonate, or magnesium stearate. The composition can be formulated as a suppository, with traditional binders and carriers such as triglycerides.

The amount of a modified aerolysin protein, such as SEQ ID NO: 6, effective in the treatment of a particular disorder or condition, such as lung cancer, will depend on the nature of the disorder or condition, and can be determined by standard clinical techniques. In addition, *in vitro* assays can be employed to identify optimal dosage ranges. The precise dose to be employed in the formulation will also depend on the seriousness of the disease or disorder, and should be decided according to the judgment of the practitioner and each subject's circumstances. Effective doses can be extrapolated from dose-response curves derived from *in vitro* or animal model test systems. Examples of effective iv doses of a modified aerolysin protein for a 70 kg human are about 1 µg -10 mg of a modified aerolysin protein. Examples of effective intratumor doses of a modified aerolysin protein for a 70 kg human are about 10 µg-100 mg of a modified aerolysin protein.

The disclosure also provides a pharmaceutical pack or kit comprising one or more containers filled with one or more of the ingredients of the pharmaceutical compositions. Optionally associated with such container(s) can be a notice in the form prescribed by a governmental agency regulating the manufacture, use or sale of pharmaceuticals or biological products, which notice reflects approval by the agency of manufacture, use or sale for human administration. Instructions for use of the composition can also be included.

Having illustrated and described novel modified aerolysin molecules, and methods of using these molecules for treating lung cancer and metastases, it should be apparent to one skilled in the art that the disclosure can be modified in arrangement and detail without departing from such principles. In view of the many possible embodiments to which the principles of our disclosure may be applied, it should be recognized that the illustrated embodiments are only particular examples of the disclosure and should not be taken as a limitation on the scope of the disclosure. Rather, the scope of the disclosure is in accord with the following claims. We therefore claim as our invention all that comes within the scope and spirit of these claims.

## Claims

1. A purified peptide comprising:
a lung cancer-specific binding agent, wherein the lung cancer-specific binding agent
comprises a lung cancer-specific antibody.

2. The purified peptide of claim 1, wherein the lung cancer-specific antibody comprises AFAI.

3. The purified peptide according to any of the previous claims, wherein the AFAI comprises at least 80% identity to amino acids 1-135 of SEQ ID NO: 6.

4. The purified peptide according to any of the previous claims, wherein the AFAI comprises amino acids 1-135 of SEQ ID NO: 6 or variants as long as such variant retains the ability to target a toxin to a lung cancer cell.

5. The purified peptide according to any of the previous claims, wherein AFAI is linked to a toxin.

6. The purified peptide according to any of the previous claims, wherein the purified peptide comprises at least 95% sequence identity to amino acid 1-135 of SEQ ID NO: 6, and retains the ability to binding a lung tumor cell and reduce lung tumor cell volume.

7. The purified peptide, wherein the purified peptide comprises one or more amino acid substitutions.

8. An isolated nucleic acid molecule, comprising a nucleic acid sequence that encodes the purified peptide according to any of the previous claims.

9. The isolated nucleic acid molecule according to claim 8, wherein the nucleic acid sequence is operably linked to a promoter sequence.

10. The isolated nucleic acid molecule according to any of the previous claims 8 or 9, wherein the sequence comprises at least 95% sequence identity to nucleic acid No. 1 to 105 of SEQ ID NO: 5.

11. The isolated nucleic acid molecule according to any of the previous claims 8 to 10, wherein the nucleic acid sequence includes one or more substitutions that result in one or more amino acid substitutions.

12. The isolated nucleic acid molecule according to any of the previous claims 8 to 11, wherein the nucleic acid sequence includes one or more substitutions, which results in no more than 10 amino acid substitutions.
